# EUROPEAN PATENT APPLICATION

(11) **EP 1 627 632 A1**
(43) Date of publication of application: **22.02.2006**
(21) Application number: 04733687.0
(22) Date of filing: 18.05.2004
(51) Int. Cl.: A61K 31/11, A61P 3/04, A61P 3/10, A23L 1/30, A23K 1/16

(54) **THERAPEUTIC AGENT**

(30) Priority: 19.05.2003 JP 2003140811; 10.10.2003 JP 2003351943
(71) Applicant: TAKARA BIO INC., Otsu-shi, Shiga 520-2193 (JP)
(72) Inventor: ENOKI, Tatsuji, Kyotanabe-shi, Kyoto 610-0313 (JP); KOBAYASHI, Eiji, Otsu-shi, Shiga 520-2153 (JP); OGAWA, Kinuko, Otsu-shi, Shiga 520-2134 (JP); KUDO, Yoko, Otsu-shi, Shiga 520-2134 (JP); TANABE, Masashige, Otsu-shi, Shiga 520-2133 (JP); OHNOGI, Hiromu, Kusatsu-shi, Shiga 525-0025 (JP); SAGAWA, Hiroaki, Kusatsu-shi, Shiga 525-0025 (JP); KATO, Ikunoshin, Koka-shi, Shiga 529-1851 (JP)
(74) Representative: Vossius & Partner
(86) International application number: PCT/JP2004/007041
(87) International publication number: WO 2004/100936

(57) **Abstract**

The present invention relates to a therapeutic agent or prophylactic agent for a disease accompanying an abnormality in an amount of insulin or insulin response, an insulin-mimetic action agent, a food, beverage, or feed, an agent for enhancement of glucose uptake into a cell, and an agent for inducing differentiation into an adipocyte, each comprising as an effective ingredient at least one compound selected from the group consisting of specified compounds having an insulin-mimetic action, derivatives thereof, and pharmacologically acceptable salts thereof.

## Description

### TECHNICAL FIELD

The present invention relates to a medicament, food, beverage or feed which is useful in treating or preventing a disease associated with insulin in a living body, for example, diabetes, obesity or the like.

### BACKGROUND ART

Insulin is a hormone necessary for normal metabolism of carbohydrates, proteins and fats in a mammal. Since human suffering from type I diabetes does not sufficiently produce insulin, which is a hormone sustaining life, administration of insulin from the external is required for survival. Human suffering from type II diabetes needs administering with insulin or an agent for enhancing insulin secretion in order to control the glucose level in blood from an inappropriate level, due to causations such as deficiency of the amount of insulin produced and insulin resistance, to an appropriate level. However, among humans suffering from type II diabetes, therapeutic effects may not be found in cases where insulin or the agent for enhancing insulin secretion is administered to diabetic patients of which cause is insulin resistance caused by hyperinsulinemia, abnormality in insulin receptor, aberrance in a downstream signal of the insulin receptor or the like.

In recent years, in order to solve the side effects of insulin and the above-mentioned problems, developments have been made on a substance having physiological functions similar to those of insulin (hereinafter referred to as insulin-mimetic substance in some cases). It has been found that a synthetic benzoquinone derivative is an insulin-mimetic substance (for example, WO 99/51225), and that shikonin derived from *Lithospermum erythrorhizon* is an insulin-mimetic substance (for example, Kamei R. and seven others, *Biochem. Biophys. Res. Commun.,* 2002, Vol. 292, P642-651). The insulin-mimetic substances as mentioned above have been expected to ameliorate symptoms by exhibiting physiological activities similar to those of insulin, not only in type I diabetic patients but also in type II diabetic patients, and even more in type II diabetic patients of which cause is insulin resistance.

Since polygodial, a sesquiterpene having dialdehyde, is contained in *Polygonum hydropiper* or the like and has a hot taste, the polygodial has been utilized as a spice from old times. As pharmacological actions of the polygodial, an anti-trichophytic action (for example, Japanese Patent Laid-Open No. Sho 63-156718), an anti-tumor action (for example, Japanese Patent Laid-Open No. Hei 3-25119), a fungicidal action (for example, Japanese Patent Laid-Open No. Hei 7-135942, Japanese Patent Laid-Open No. Hei 7-285820) and the like have been known. However, an insulin-mimetic action such as an anti-diabetic action or anti-obesity action has not been known so far.

### DISCLOSURE OF INVENTION

An object of the present invention is to develop a substance having insulin-mimetic action suitable as food materials and medicament materials, which is capable of being conveniently taken, and to provide a medicament, food, beverage or feed using the composition or substance.

Summarizing the present invention, a first invention of the present invention relates to a therapeutic agent or prophylactic agent for a disease accompanying an abnormality in an amount of insulin or insulin response, characterized in that the therapeutic agent or prophylactic agent comprises as an effective ingredient at least one compound selected from the group consisting of a compound represented by the following general formula (Formula 1): 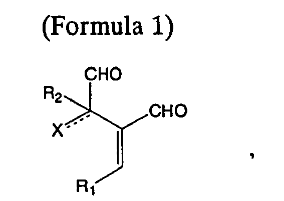
wherein a bond containing a dotted line is a single bond or a double bond; each of R₁ and R₂, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue; and X is a hydrogen atom or a carbon atom; in a case where X is a carbon atom, to the carbon atom may be added a hydrogen atom, a halogen group, an acyl group, an amino group, a nitro group, a hydroperoxy group, an aliphatic group, an aromatic group, an aromatic aliphatic group, or a sugar residue; or in a case where R₁ is an aliphatic group, X and R₁ may together form a 5- to 9-membered ring, and the 5- to 9-membered ring may have a resonance structure; a polycyclic structure comprising one or more 4- to 6-membered rings may be further formed adjacent to the 5- to 9-membered ring as long as a structure of a part excluding X and R₁ in the general formula (Formula 1) is maintained, wherein at least one substituent selected from a hydroxyl group which may be esterified or etherified, a halogen group, an acyl group, an amino group, a nitro group, a hydroperoxy group, an aliphatic group, an aromatic group, an aromatic aliphatic group and a sugar residue may be added to the polycyclic group; a ketone structure and/or an epoxy structure may be contained in the molecule; in a case where X and R₁ together form a 6-membered ring, if R₂ is the aliphatic group, X and R₂ may further together form a 3-membered ring;
a compound represented by the following general formula (Formula 2): 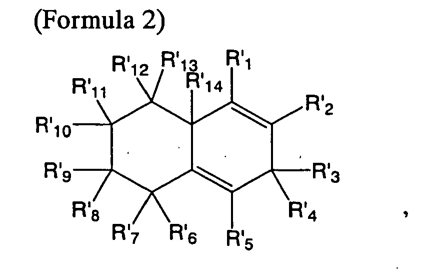
wherein each of R'₁ to R'₁₄, which may be identical or different, is a hydrogen atom, a halogen group, an acyl group, a hydroxyl group which may be esterified or etherified, an amino group, a nitro group, a hydroperoxy group, an aliphatic group, an aromatic group, an aromatic aliphatic group, or a sugar residue, or one or more pairs selected from R'₁ and R'₂, R'₂ and R'₃, R'₃ and R'₄, R'₄ and R'₅, R'₅ and R'₆, R'₆ and R'₇, R'₇ and R'₈, R'₈ and R'₉, R'₉ and R'₁₀, R'₁₀ and R'₁₁, R'₁₁ and R'₁₂, R'₁₂ and R'₁₃, R'₁₃ and R'₁₄, and R'₁₄ and R'₁ may form a ring containing one or more atoms selected from a carbon atom, an oxygen atom, a nitrogen atom and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be further bound to the above ring; or one or more pairs selected from R₃' and R'₄, R'₆ and R'₇, R'₈ and R'₉, R'₁₀ and R'₁₁, and R'₁₂ and R'₁₃ may together form a ketone structure with a carbon atom in the ring to which the pairs are bound; a compound represented by the following general formula (Formula 3): 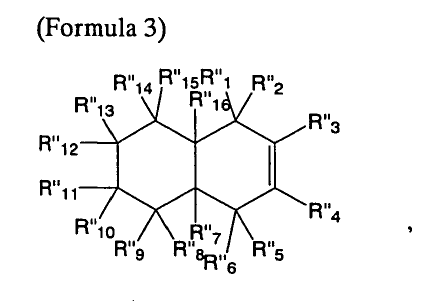
wherein each of R"₁ to R"₁₆, which may be identical or different, is a hydrogen atom, a halogen group, an acyl group, a hydroxyl group which may be esterified or etherified, an amino group, a nitro group, a hydroperoxy group, an aliphatic group, an aromatic group, an aromatic aliphatic group, or a sugar residue, or one or more pairs selected from R"₁ and R"₂, R"₂ and R"₃, R"₃ and R"₄, R"₄ and R"₅, R"₅ and R"₆, R"₆ and R"₇, R"₇ and R"₈, R"₈ and R"₉, R"₉ and R"₁₀, R"₁₀ and R"₁₁, R"₁₁ and R"₁₂, R"₁₂ and R"₁₃, R"₁₃ and R"₁₄, R"₁₄ and R"₁₅, R"₁₅ and R"₁₆, and R"₁₆ and R"₁ may form a ring containing one or more atoms selected from a carbon atom, an oxygen atom, a nitrogen atom and a sulfur atom in a possible range, wherein a halogen group, an acyl group, a hydroxyl group which may be esterified or etherified, an amino group, a nitro group, a hydroperoxy group, an aliphatic group, an aromatic group, an aromatic aliphatic group, or a sugar residue may be further bound to the above ring; or one or more pairs selected from R"₁ and R"₂, R"₅ and R"₆, R"₈ and R"₉, R"₁₀ and R"₁₁, R"₁₂ and R"₁₃, and R''₁₄ and R''₁₅ may together form a ketone structure with a carbon atom in the ring to which the pairs are bound; derivatives thereof; and pharmacologically acceptable salts thereof.

As the compound represented by the above-mentioned general formula (Formula 1), for example, at least one compound selected from the group consisting of a compound represented by the following general formula (Formula 4): 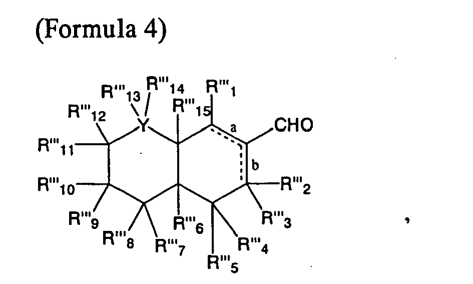
wherein Y is 0 or 1 carbon atom, and in a case where Y is 0 carbon atom, R'''₁₃ and R'''₁₄ are not present; each of R'''₁ and R'''₂, which is different from each other, is a hydrogen atom or an aldehyde group, and in a case where R'''₁ is an aldehyde group, a bond **a** is a single bond, and a bond **b** is a double bond; or in a case where R'''₂ is an aldehyde group, a bond **a** is a double bond, and a bond **b** is a single bond; each of R'''₃ to R'''₁₅, which may be identical or different, is a hydrogen atom, a halogen group, an acyl group, a hydroxyl group which may be esterified or etherified, or a saturated or unsaturated hydrocarbon group having 1 to 10 carbon atoms, with proviso that R'''₃ may not be present in some cases; or R'''₃ and R'''₄ may together form a 3-membered ring with a carbon atom in the ring in which these groups are bound to each other; or one or more pairs selected from R'''₄ and R'''₅, R'''₇ and R'''₈, and R'''₉ and R'''₁₀, R'''₁₁ and R'''₁₂, and R'''₁₃ and **R'''**_{**14**} **may** together form a ketone structure and/or a 3-membered epoxy ring structure with a carbon atom in the ring to which the pairs are bound; a compound represented by the following general formula (Formula 5): 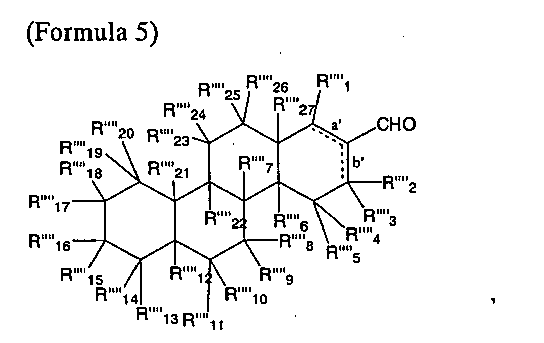
wherein each of R''''₁ and R''''₂, which is different from each other, is a hydrogen atom or an aldehyde group, and in a case where R''''₁ is an aldehyde group, a bond **a'** is a single bond, and a bond **b'** is a double bond; or in a case where R''''₂ is an aldehyde group, a bond **a'** is a double bond, and a bond **b'** is a single bond; each of R''''₃ to R''''₂₇, which may be identical or different, is a hydrogen atom, a halogen group, an acyl group, a hydroxyl group which may be esterified or etherified, or a saturated or unsaturated hydrocarbon group having 1 to 10 carbon atoms, with proviso that R''''₃ may not be present in some cases; or R''''₃ and R''''₄ may together form a 3-membered ring with a carbon atom in the ring in which these groups are bound to each other, or one or more pairs selected from R''''₄ and R''''₅, R''''₈ and R''''₉, R''''₁₀ and R''''₁₁, R''''₁₃ and R''''₁₄, R''''₁₅ and R''''₁₆, R''''₁₇ and R''''₁₈, R''''₁₉ and R''''₂₀, R''''₂₃ and R''''₂₄, and R''''₂₅ and R''''₂₆ may together form a ketone structure and/or form a 3-membered epoxy ring structure with a carbon atom in the above ring to which the pairs are bound; and a compound represented by the following general formula (Formula 6): 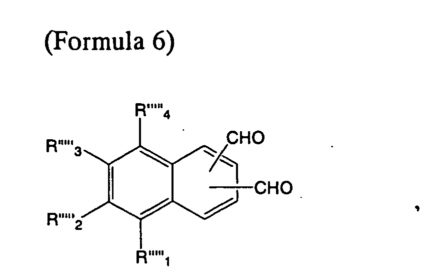
wherein each of R'''''₁ to R'''''₄, which may be identical or different, is a hydrogen atom, a halogen group, an acyl group, a hydroxyl group which may be esterified or etherified, or a saturated or unsaturated hydrocarbon group having 1 to 10 carbon atoms, wherein the two aldehyde groups attached to the ring on the right are added to adjacent hydrocarbon atoms in the ring is preferable.

As the compound represented by the above-mentioned general formula (Formula 2), a compound in which each of R'₁, R'₂, R'₆, R'₇ and R'₁₄ in the compound is a methyl group, an aldehyde group, a carboxyl group, a methoxycarbonyl group, an acetoxymethyl group or a hydroxymethyl group is preferable. As the compound represented by the above-mentioned general formula (Formula 3), a compound in which each of R"₁, R"₃, R"₈, R"₉ and R"₁₆ in the compound is a methyl group, an aldehyde group, a carboxyl group, a methoxycarbonyl group, an acetoxymethyl group or a hydroxymethyl group is preferable.

As the compound represented by the above-mentioned general formula (Formula 2), also preferable is a compound represented by the following general formula (Formula 7): 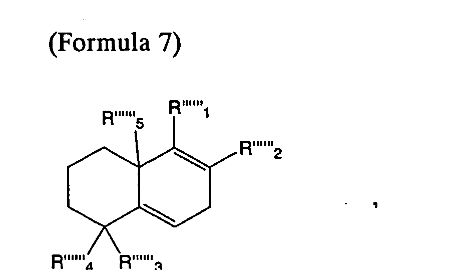
wherein each of R'''''₁ to R'''''₅, which may be identical or different, is a methyl group, an aldehyde group, a carboxyl group, a methoxycarbonyl group, an acetoxymethyl group, or a hydroxymethyl group.

As the compound represented by the above-mentioned general formula (Formula 3), also preferable is a compound represented by the following general formula (Formula 8): 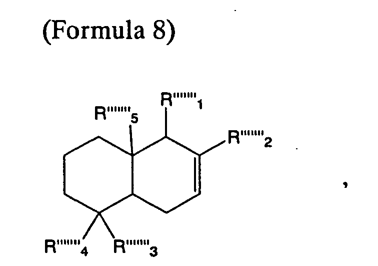
wherein each of R'''''₁ to R'''''₅, which may be identical or different, is a methyl group, an aldehyde group, a carboxyl group, a methoxycarbonyl group, an acetoxymethyl group, or a hydroxymethyl group.

Further, as the compound represented by the above-mentioned general formula (Formula 1), at least one compound selected from the group consisting of polygodial, epipolygodial, isovelleral, 12-epi-scalaradial, naphthalene-2,3-dicarboxyaldehyde, and miogadial is preferable. As the compound represented by the above-mentioned general formula (Formula 2), at least one compound selected from the group consisting of dimethyl 3,5,6,7,8,8a-hexahydro-5,5,8a-trimethyl-1,2-naphthalenedicarboxylate, 3,5,6,7,8,8a-hexahydro-5,5,8a-trimethyl-1,2-naphthalenedimethanol, and 4,6,7,8,9,9a-hexahydro-6,6,9a-trimethyl-naphtho[1,2-c]furan-1(3H)-one is preferable. As the compound represented by the above-mentioned general formula (Formula 3), at least one compound selected from the group consisting of dimethyl 1,4,4a,5,6,7,8,8a-octahydro-5,5,8a-trimethyl-1,2-naphthalenedicarboxylate, 1,4,4a,5,6,7,8,8a-octahydro-5,5,8a-trimethyl-1,2-naphthalenedimethanol, 5,5a,6,7,8,9,9a,9b-octahydro-6,6,9a-trimethyl-naphtho[1,2-c]furan-3(1H)-one, 1-[(acetyloxy)methyl]-1,4,4a,5,6,7,8,8a-octahydro-5,5,8a-trimethyl-2-naphthalenecarboxyaldehyde, and 2-[(acetyloxy)methyl]-1,4,4a,5,6,7,8,8a-octahydro-5,5,8a-trimethyl-1-naphthalenecarboxyaldehyde is preferable.

Second to fifth inventions of the present invention relate to an agent for an insulin-mimetic action, a food, beverage or feed for treating or preventing a disease accompanying an abnormality in an amount of insulin or insulin response, an agent for enhancing glucose uptake into a cell, and an agent for inducing differentiation into an adipocyte, characterized in that each comprises an effective ingredient of the first invention of the present invention.

A sixth invention of the present invention relates to a novel compound 3,5,6,7,8,8a-hexahydro-5,5,8a-trimethyl-1,2-naphthalenedimethanol or a salt thereof.

According to the present invention, there is provided a medicament, food, beverage, feed or the like, for treating or preventing a disease accompanying an abnormality in an amount of insulin or insulin response. The medicament is useful as a therapeutic agent or prophylactic agent for a disease accompanying an abnormality in an amount of insulin or insulin response such as diabetes or obesity. Also, according to the food or beverage, by taking it as foodstuff on a daily basis, symptoms of a disease accompanying an abnormality in an amount of insulin or insulin response can be ameliorated and the like. Therefore, the foodstuff containing the compound used in the present invention can be said to be functional foodstuff, and are useful in maintaining homeostasis of a living body due to their insulin-mimetic actions. In addition, the feed of the present invention can also be expected to exhibit similar effects.

### BRIEF DESCRIPTION OF THE DRAWINGS

Figure 1 is a graph showing an enhancing action of polygodial on glucose uptake.
Figure 2 is a graph showing an enhancing action of 12-epi-scalaradial on glucose uptake.
Figure 3 is a graph showing an enhancing activity of naphthalene-2,3-dicarboxyaldehyde on glucose uptake.
Figure 4 is a chart showing ¹H-NMR spectrum of a compound (P001).
Figure 5 is a graph showing an action for inducing differentiation into an adipocyte by a compound (P001).
Figure 6 is a chart showing ¹H-NMR spectrum of a compound (P002).
Figure 7 is a graph showing an action for inducing differentiation into an adipocyte by a compound (P002).
Figure 8 is a chart showing ¹H-NMR spectrum of a compound (P003).
Figure 9 is a graph showing an action for inducing differentiation into an adipocyte by a compound (P003).
Figure 10 is a chart showing ¹H-NMR spectrum of a compound (P004).
Figure 11 is a graph showing an enhancing action of a compound (P004) on glucose uptake.
Figure 12 is a graph showing an action for inducing differentiation into an adipocyte by a compound (P004).
Figure 13 is a chart showing ¹H-NMR spectrum of a compound (P005).
Figure 14 is a graph showing an action for inducing differentiation into an adipocyte by a compound (P005).
Figure 15 is a chart showing ¹H-NMR spectrum of a compound (P006).
Figure 16 is a graph showing an action for inducing differentiation into an adipocyte by a compound (P006).
Figure 17 is a chart showing ¹H-NMR spectrum of a compound (P007).
Figure 18 is a graph showing an action for inducing differentiation into an adipocyte by a compound (P007).
Figure 19 is a chart showing ¹H-NMR spectrum of a compound (P008).
Figure 20 is a graph showing an action for inducing differentiation into an adipocyte by a compound (P008).
Figure 21 is a graph showing a synergistic action of polygodial and insulin to enhance glucose uptake.
Figure 22 is a graph showing an enhancing action of 4-hydroxyderricin on glucose uptake inhibited by cytochalasin B.
Figure 23 is a graph showing an enhancing action of a compound (P003) on glucose uptake.
Figure 24 is a graph showing an enhancing action of isovelleral on glucose uptake.
Figure 25 is a graph showing an enhancing action of epipolygodial on glucose uptake.

### BEST MODE FOR CARRYING OUT THE INVENTION

The medicament, food, beverage, feed or the like provided by the present invention comprises as an effective ingredient at least one compound selected from the group consisting of a compound represented by the above general formula (Formula 1), a compound represented by the above general formula (Formula 2), a compound represented by the above general formula (Formula 3), derivatives thereof, and pharmacologically acceptable salts thereof. The desired effects of the present invention as described below are exhibited based on the insulin-mimetic action exhibited by these effective ingredients.

In the present invention, the insulin-mimetic action is not particularly limited as long as at least one of the physiological activities possessed by insulin is exhibited. For example, the insulin-mimetic action is exemplified by metabolic regulatory actions such as enhancement of uptake of a sugar or an amino acid in a cell, and synthesis and degradation inhibition of glycogen or protein. Also, the presence or absence of the insulin-mimetic action can be conveniently determined in accordance with the method described in Example 1 or 5 set forth below.

The halogen group as used herein is not particularly limited. The halogen group includes, for example, a fluoro group, a chloro group, a bromo group, an iodo group, an iodosyl group, an iodyl group, a dichloroiodo group, and the like.

The acyl group is not particularly limited. The acyl group includes, for example, an aldehyde group, a carboxyl group, a methoxycarbonyl group, an acetyl group, an aroyl group and the like.

The hydroxyl group which may be esterified or etherified is not particularly limited. The hydroxyl group includes, for example, a hydroxyl group, a methoxy group, an ethoxy group, an acetoxy group, a benzyloxy group, a tetrahydropyranyloxy group, a prenyloxy group, a geranyloxy group, a farnesyloxy group, an ethylcarbonyloxy group, a propylcarbonyloxy group, a butylcarbonyloxy group, a benzylcarbonyloxy group, a cyclohexenylcarbonyloxy group, a 2,4,6-octatrienoyloxy group, a p-coumaroyloxy group, a 2-acetoxydecanoyloxy group, a 2-hydroxydecanoyloxy group, a 2-hydroxyoctanoyloxy group, a 2-hydroxynonanoyloxy group, a 3-hydroxybutanoyloxy group, a 2-hexenoyloxy group and the like.

The aliphatic group refers to a saturated or unsaturated, linear, branched or cyclic hydrocarbon group to which an optional functional group (including a substituent) may be added. The hydrocarbon group used herein is not particularly limited. The preferred hydrocarbon group is exemplified by a linear alkyl group, a branched alkyl group, a linear alkenyl group, a branched alkenyl group, a cycloalkyl group and the like, each having 1 to 30 carbon atoms. Further, the aliphatic group as used herein also encompasses those prepared by adding the hydroxyl group which may be esterified or etherified, the halogen group, the acyl group, the amino group, the nitro group or the hydroperoxy group mentioned above to these aliphatic groups; and these aliphatic groups containing an epoxy structure or a ketone structure in the molecules.

Specifically, the aliphatic group as used herein is exemplified by, for example, a methyl group, a methylene group (CH₂=), a carboxymethyl group, an acetoxymethyl group, a hydroxymethyl group, an ethyl group, an ethylene group, an n-propyl group, an isopropyl group, an isobutyl group, a sec-butyl group, a tert-butyl group, an isopentyl group, a neopentyl group, a tert-pentyl group, an ethenyl group, an allyl group, a trans-1-propenyl group, a cis-1-propenyl group, a methylbutyl group, a prenyl group, an isohexenyl group, a geranyl group, a farnesyl group, an isopropenyl group, a cis-1-methyl-1-propenyl group, a trans-1-methyl-1-propenyl group, a trans-1-methyl-1-propenyl group, a trans-1-ethyl-1-propenyl group, an adamantyl group, a (2,2-dimethyl-6-methylenecyclohexyl)methyl group, a (2,6,6-trimethyl-2-cyclohexenyl)methyl group, a 1,5-dimethyl-4-hexenyl group, a 1-(acetoxymethyl)-5-methyl-4-hexenyl group, a 1-formyl-2-(2,6-dimethyl-1,5-heptadienyl)-cyclopropyl group, a 1-formylvinyl group, a 1,4-dimethyl-3-hexenyl group, a 4-hydroxy-4-methyl-2-pentenyl group, a 5-hydroxy-4-methyl-3-hexenyl group, a 3,4-dimethyl-4-pentenyl group, a 2-methylene-5,5,8a-trimethylperhydro-1-naphthylmethyl group, a 2,2-(epoxymethano)-5,5,8a-trimethylperhydro-1-naphthylmethyl group, a 1,2,4a,5-tetramethyl-1,2,3,4,4a,7,8,8a-octahydro-1-naphthylmethyl group, a 5-methylene-1,2,4a-trimethylperhydro-1-naphthylmethyl group, a 7-hydroxy-3,7-dimethyl-1,3,5-octatrienyl group, and a 3,7-dimethyl-2,6-octadien-4-ynyl group.

When the aliphatic group is a methylene group (CH₂=), two functional groups attached to the same carbon atom together form the group.

The saturated or unsaturated hydrocarbon group having 1 to 10 carbon atoms in the above-mentioned general formulas (Formula 4) to (Formula 6) is not particularly limited. The hydrocarbon group is exemplified by, for example, a methyl group, a methylene group (CH₂=), an ethyl group, an ethylene group, a propyl group, a butyl group, a pentyl group, a prenyl group, an isohexenyl group, a methylbutyl group, a geranyl group, a 1,4-dimethyl-3-hexenyl group, a 4-hydroxy-4-methyl-2-pentenyl group, a 5-hydroxy-4-methyl-3-hexenyl group, a 3,4-dimethyl-4-pentenyl group, and the like.

In addition, the aromatic group includes, for example, a phenyl group, a furyl group, a thienyl group, a naphthyl group, a biphenyl group, and a pyrrolyl group, a pyridyl group, an indolyl group, an imidazolyl group, a tolyl group, a xylyl group, and the like. Also, the aromatic group as used herein encompasses those compounds prepared by adding a hydroxyl group which may be esterified or etherified, a halogen group, an acyl group, an amino group, a nitro group, a hydroperoxy group or the like to these aromatic groups.

The aromatic aliphatic group is exemplified by aromatic aliphatic groups having a saturated or unsaturated, linear or branched hydrocarbon group having 1 to 20 carbon atoms (aliphatic moiety), and the aromatic aliphatic group includes, for example, a phenylalkyl group of which alkyl group has 1 to 20 carbon atoms (e.g., a benzyl group, a phenethyl group), a 2-phenylvinyl group, a 2-(4-hydroxyphenyl)vinyl group, a 2-(2-furyl)vinyl group, a 2-(2-thienyl)vinyl group, a 4-phenyl-1,3-butadienyl group, a styryl group, a cinnamyl group and the like. In addition, the aromatic aliphatic group as used herein also encompasses those prepared by adding a hydroxyl group which may be esterified or etherified, a halogen group, an acyl group, a nitro group, an amino group, or a hydroperoxy group to these aromatic aliphatic groups.

The sugar constituting the sugar residue includes, for example, monosaccharides such as glucose, threose, ribose, apiose, allose, rhamnose, arabinopyranose, ribulose, xylose, galactose, 3,6-anhydrogalactose, mannose, talose, fucose, fructose, glucuronic acid, and galacturonic acid; disaccharides such as gentiobiose, neohesperidose, rutinose, agarobiose, isomaltose, sucrose, xylobiose, nigerose, maltose, and lactose; an oligosaccharide derived from a polysaccharide such as agarose, fucoidan, and starch; polysaccharides such as agarose, fucoidan, and starch; and the like. In addition, the sugar residue includes a compound in which a sugar is O-, N-, S-, or C-glycoside-bound, and a compound in which a sugar is bound via a C-C bond to a carbon other than a reducing end of a sugar. In addition, the sugar residue as used herein also encompasses those prepared by adding a hydroxyl group which may be esterified or etherified, a halogen group, an acyl group, a nitro group, an amino acid, a hydroperoxy group, a sulfate group, a phosphate group or the like to these sugar residues.

The compound represented by the above-mentioned general formula (Formula 1) of the present invention encompasses a compound in which X and R₁ together form a 5- to 9-membered ring, and further form a polycyclic structure comprising one or more 4- to 6-membered rings, adjoining the 5- to 9-membered ring. When the 5- to 9-membered ring has a resonance structure, for example, the case of a benzene ring, the above-mentioned general formula (Formula 1) is also satisfied.

In the present specification, the phrase "within a possible range" in the explanation of the general formulas means within a range that the compound can be naturally present, or that the compound can be artificially synthesized.

In the present invention, the compound represented by the above-mentioned general formula (Formula 1) is preferably exemplified by compounds containing one to five 5-membered rings or 6-membered rings in the structure, more preferably exemplified by compounds represented by the above-mentioned general formulas (Formula 4) to (Formula 6).

In the compound represented by the above-mentioned general formula (Formula 4), in the case where the compound contains a ketone structure or a 3-membered epoxy ring structure, it is preferable that R'''₇ and R'''₈, and/or R'''₁₃ and R'''₁₄ together form the ketone structure or the epoxy structure with a carbon atom in the ring to which the groups are bound.

In the compound represented by the above-mentioned general formula (Formula 5), in the case where the compound contains a ketone structure or a 3-membered epoxy ring structure, it is preferable that R''''₂₅ and R''''₂₆ together form the ketone structure or the epoxy structure with a carbon atom in the ring to which the groups are bound.

In the above-mentioned general formula (Formula 4), especially preferably exemplified are compounds in which Y is one carbon atom, R'''₁ is an aldehyde group, R'''₂ is a hydrogen atom, the bond **a** is a single bond, and the bond **b** is a double bond; or compounds in which Y is 0 carbon atom, R'''₁ is a hydrogen atom, R'''₂ is an aldehyde group, the bond **a** is a double bond, and the bond **b** is a single bond. In addition, exemplified are compounds in which R'''₁ is a hydrogen atom, R'''₂ is an aldehyde group, the bond **a** is a double bond, the bond **b** is a single bond, and R'''₃ and **R'''**_{**4**} together form a 3-membered ring with a carbon atom in the ring to which the groups are bound.

In addition, in the above-mentioned general formula (Formula 5), especially preferably exemplified is a compound in which R''''₁ is an aldehyde group, R''''₂ is a hydrogen atom, **a** bond **a'** is a single bond, and **a** bond **b'** is a double bond.

Since the present inventors have found that the compound represented by the above-mentioned general formula (Formula 1) has an especially potent enhancing action on glucose uptake into a cell, the compound represented by the above-mentioned general formula (Formula 1) is the most preferable embodiment of the compounds used in the present invention as an effective ingredient.

Also, in the present invention, the compound represented by the above-mentioned general formula (Formula 1) is exemplified by compounds represented by the following formulas (Formula 9) to (Formula 13): 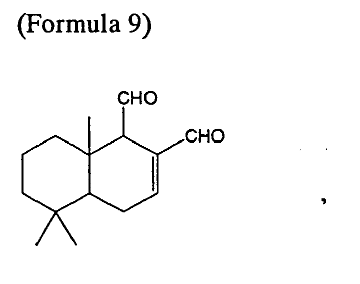 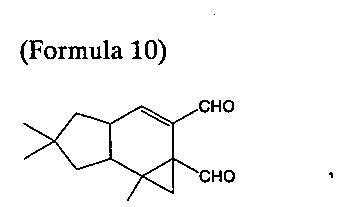 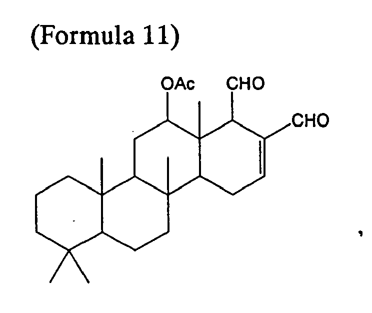 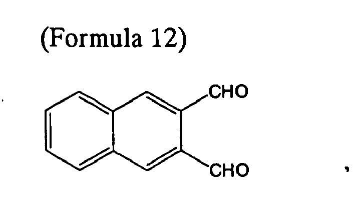 and

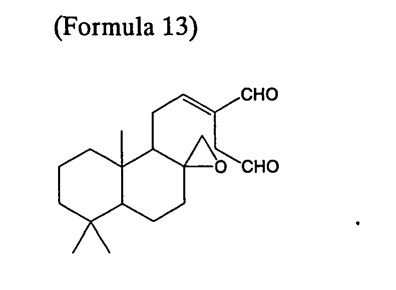

The compound represented by the above-mentioned formula (Formula 9) has two optical isomers (polygodial and epipolygodial), and both the isomers can be used in the present invention. The polygodial can be obtained by subjecting a compound represented by the following formula (Formula 21) to oxidization (Swern oxidation) with oxalyl chloride and dimethyl sulfoxide in dichloromethane. In addition, the epipolygodial can be obtained by heat-treating polygodial together with tetrahydroxyfuran in the presence of anhydrous potassium carbonate. Further, the polygodial and the epipolygodial can be obtained, for example, by extraction and purification from *Polygonum hydropiper* or the like according to a conventional method.

In addition, the compound (isovelleral) represented by above-mentioned formula (Formula 10) can be obtained, for example, by extraction and purification from a mushroom belonging to the genus *Lactarius,* for example, *Lactarius vellereus,* according to a conventional method.

The compound (12-epi-scalaradial) represented by the above-mentioned formula (Formula 11) can be obtained by extraction and purification from a poriferan (for example, *Cacospongia mollior)* according to a conventional method.

In addition, the compound (miogadial) represented by the above-mentioned formula (Formula 13) can be obtained, for example, by extraction and purification from a plant belonging to Zingiberaceae, for example, *Zingiber mioga* according to a conventional method.

In addition, as the compound represented by the above-mentioned formula (Formula 9), the compound represented by the above-mentioned formula (Formula 10), the compound represented by the above-mentioned formula (Formula 11), and the compound (naphthalene-2,3-dicarboxyaldehyde) represented by the above-mentioned formula (Formula 12), commercially available compounds can be used as they are.

In the present invention, the compound represented by the above-mentioned general formula (Formula 2) is exemplified by, for example, compounds in which each of R'₁, R'₂, R'₆, R'₇ and R'₁₄ is one member selected from a methyl group, an aldehyde group, a carboxyl group, an acetoxymethyl group, a hydroxymethyl group, and a methoxycarbonyl group in the formula (Formula 2), especially preferably exemplified by the compound represented by the above-mentioned general formula (Formula 7). In the above-mentioned general formula (Formula 7), especially preferably exemplified are compounds in which all of R''''''₃, R''''''₄ and R''''''₅ are methyl groups.

In addition, in the present invention, the compound represented by the above-mentioned general formula (Formula 2) is exemplified by compounds represented by the following formulas (Formula 14) to (Formula 18): 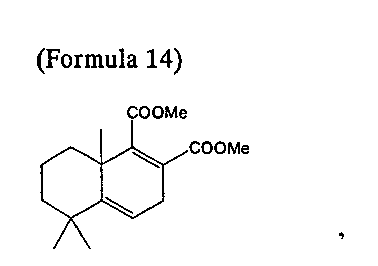 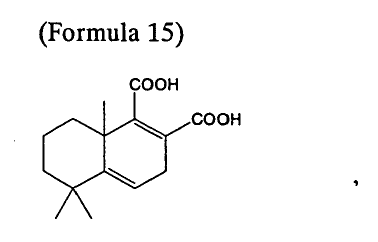 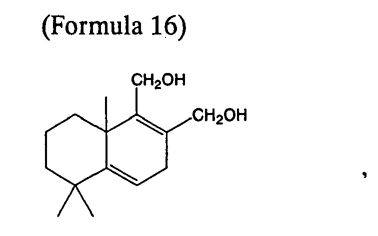 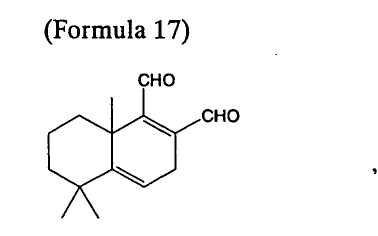 and

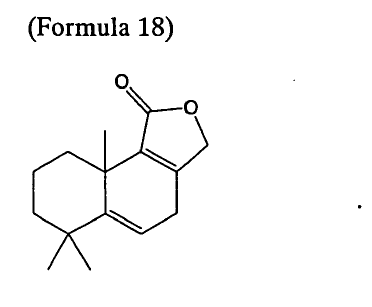

For example, the compound represented by the above-mentioned formula (Formula 14), i.e., dimethyl 3,5,6,7,8,8a-hexahydro-5,5,8a-trimethyl-1,2-naphthalenedicarboxylate can be obtained by reacting 1-vinyl-2,6,6-trimethyl-1-cyclohexene and dimethylacetylene dicarboxylate at 110°C. In addition, the compound represented by the above-mentioned formula (Formula 14) may be referred to herein as a compound (P001) in some cases.

The compound represented by the above-mentioned formula (Formula 15) can be obtained by treating the compound represented by the above-mentioned formula (Formula 14) with sodium methoxide in methanol.

The compound represented by the above-mentioned formula (Formula 16), i.e., 3,5,6,7,8,8a-hexahydro-5,5,8a-trimethyl-1,2-naphthalenedimethanol, can be obtained by treating the compound represented by the above-mentioned formula (Formula 14) with lithium aluminum hydride in anhydrous ether. Here, the compound represented by the above-mentioned formula (Formula 16) may be referred to herein as a compound (P002) in some cases.

The compound represented by the above-mentioned formula (Formula 17) can be obtained by subjecting the compound represented by the above-mentioned formula (Formula 16) to oxidation (Swern oxidation) with oxalyl chloride and dimethyl sulfoxide in dichloromethane.

The compound represented by the above-mentioned formula (Formula 18), i.e., 4,6,7,8,9,9a-hexahydro-6,6,9a-trimethyl-naphtho[1,2-c]furan-1(3H)-one, can be obtained by reacting the compound represented by the above-mentioned formula (Formula 16) with 15 molar equivalents of barium permanganate in dichloromethane. Here, the compound represented by the above-mentioned formula (Formula 18) may be referred to herein as a compound (P003) in some cases.

In the present invention, the compound represented by the above-mentioned general formula (Formula 3) is exemplified by, for example, compounds in which each of R"₁, R"₃, R"₈, R"₉ and R"₁₆ is one member selected from a methyl group, an aldehyde group, a carboxyl group, an acetoxymethyl group, a hydroxymethyl group, and a methoxycarbonyl group in the formula (Formula 3), and especially preferably exemplified by the compounds represented by the above-mentioned general formula (Formula 8). In the above-mentioned general formula (Formula 8), particularly preferably exemplified are compounds in which all of R'''''''₃, R'''''''₄ and R'''''''₅ are methyl groups.

In addition, in the present invention, the compound represented by the above-mentioned general formula (Formula 3) is exemplified by the compounds represented by the following formulas (Formula 19) to (Formula 25). 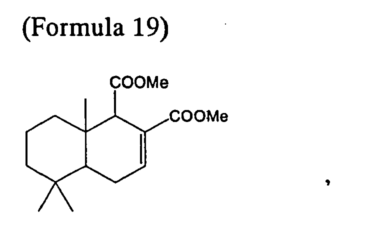 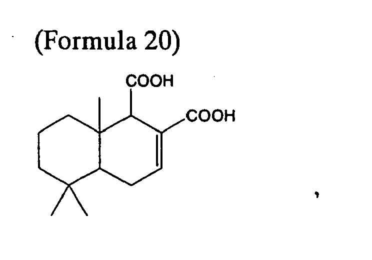 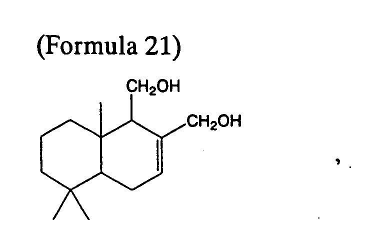 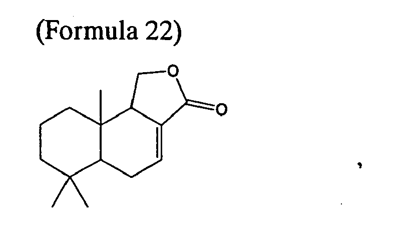 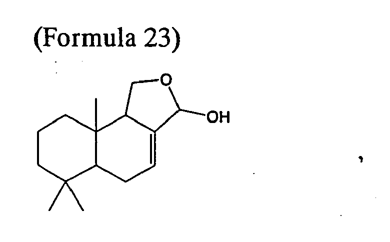 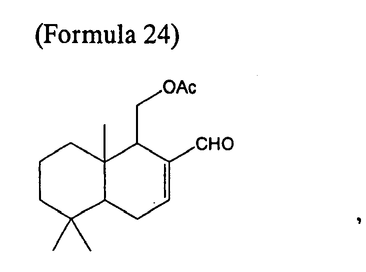 and

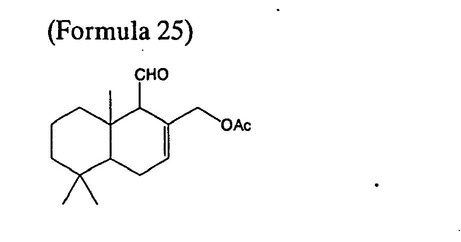

For example, the compound represented by the above-mentioned formula (Formula 19), i.e., dimethyl 1,4,4a,5,6,7,8,8a-octahydro-5,5,8a-trimethyl-1,2-naphthalenedicarboxylate, can be obtained by subjecting the compound represented by the above-mentioned formula (Formula 14) to hydrogenation under pressure (1 atm.) using palladium carbon as a catalyst in methanol containing a catalytic amount of hydrochloric acid. Here, the compound represented by the above-mentioned formula (Formula 19) may be referred to herein as a compound (P004).

The compound represented by the above-mentioned formula (Formula 20) can be obtained by treating the compound represented by the above-mentioned formula (Formula 19) with sodium methoxide in methanol.

The compound represented by the above-mentioned formula (Formula 21), i.e., 1,4,4a,5,6,7,8,8a-octahydro-5,5,8a-trimethyl-1,2-naphthalenedimethanol, can be obtained by treating the compound represented by the above-mentioned formula (Formula 19) with lithium aluminum hydride in anhydrous ether. In addition, the compound represented by the above-mentioned formula (Formula 21) may be referred to herein as a compound (P005) in some cases.

The compound represented by the above-mentioned formula (Formula 22), i.e., 5,5a,6,7,8,9,9a,9b-octahydro-6,6,9a-trimethyl-naphtho[1,2-c]furan-3(1H)-one, can be obtained by treating the compound represented by the above-mentioned formula (Formula 21) with 10 to 20 molar equivalents of barium permanganate in dichloromethane. In addition, the compound represented by the above-mentioned formula (Formula 22) may be referred to herein as a compound (P006) in some cases.

The compound represented by the above-mentioned formula (Formula 23) can be obtained by treating the compound represented by the above-mentioned formula (Formula 21) with 1 to 2 molar equivalents of barium permanganate in dichloromethane.

The compound represented by the above-mentioned formula (Formula 24), i.e., 1-[(acetyloxy)methyl]-1,4,4a,5,6,7,8,8a-octahydro-5,5,8a-trimethyl-2-naphthalenecarboxyaldehyde, can be obtained by oxidizing 1-[(acetyloxy)methyl]-1,4,4a,5,6,7,8,8a-octahydro-5,5,8a-trimethyl-1-naphthalenemethanol, which is obtained by subjecting the compound represented by the above-mentioned formula (Formula 21) to t-butyldimethylsilylation (TBDMS), acetylating the resulting product, and deprotecting the TBDMS group, with manganese dioxide. In addition, the compound represented by the above-mentioned formula (Formula 24) may be referred to as a compound (P007) in some cases.

The compound represented by the above-mentioned formula (Formula 25), i.e., 2-[(acetyloxy)methyl]-1,4,4a,5,6,7,8,8a-octahydro-5,5,8a-trimethyl-1-naphthalenecarboxyaldehyde, can be obtained by oxidizing 2-[(acetyloxy)methyl]-1,4,4a,5,6,7,8,8a-octahydro-5,5,8a-trimethyl-2-naphthalenemethanol, which is obtained by subjecting the compound represented by the above-mentioned formula (Formula 21) to t-butyldimethylsilylation (TBDMS), acetylating the resulting product, and deprotecting the TBDMS group, with pyridinium chlorochromate. In addition, the compound represented by the above-mentioned formula (Formula 25) may be referred to herein as a compound (P008) in some cases.

The compound represented by the above-mentioned formula (Formula 9), and the compound represented by the above-mentioned formula (Formula 11) are compounds that are also embraced in the above-mentioned general formula (Formula 3), and can be more preferably used in the present invention.

As the derivative of the compound used in the present invention, for example, a derivative (prodrug) which can be easily hydrolyzed in a body to exhibit the desired effects, such as an ester can be prepared. In addition, the derivative of the compound used in the present invention also encompasses a derivative obtained by adding a protecting group such as a tetrahydropyranyl group to a hydroxyl group. In addition, the derivative of the present invention encompasses a derivative obtained by administering the compound of the present invention to a mammal, to give a product via metabolism. The prodrug may be prepared in accordance with a known process. Here, the derivative may be a salt thereof.

As the salt of the compound used in the present invention or a derivative thereof, a pharmacologically acceptable salt may be used. Alternatively, the salt may be a derivative of the compound which can function as a prodrug. Therefore, the compound used in the present invention encompasses a derivative thereof and a salt thereof, as long as the desired effects of the present invention can be obtained. In addition, various isomers such as an optical isomer, a keto-enol tautomer, and a geometrical isomer of the compound used in the present invention, and isolated products of each of isomers can be all used in the present invention as long as these isomers have insulin-mimetic actions.

The pharmacologically acceptable salt of the compounds as described herein is exemplified by, for example, alkali metal salts, alkaline earth metal salts, salts with organic bases, and the like. The pharmacologically acceptable salt used in the present invention means a salt of a compound which is substantially atoxic to an organism and has an insulin-mimetic action. The salt includes, for example, salts with sodium, potassium, calcium, magnesium, ammonium, protonated benzathine (N,N'-di-benzylethylenediamine), choline, ethanolamine, diethanolamine, ethylenediamine, meglamine (N-methylglucamine), benethamine (N-benzylphenethylamine), piperazine, tolomethamine (2-amino-2-hydroxymethyl-1,3-propanediol) or the like.

The compound used in the present invention is referred to as the effective ingredient of the present invention, and a therapeutic agent or prophylactic agent for a disease accompanying an abnormality in an amount of an insulin or insulin response, which comprises the effective ingredient of the present invention may be referred to as the therapeutic agent or prophylactic agent of the present invention in some cases.

Polygodial, which is one of the effective ingredients of the present invention, can be fractionated from a plant belonging to Polygonaceae, for example, *Polygonum hydropiper* by a known method, or a fraction containing the effective ingredient in a high concentration can be also used as the effective ingredient of the present invention. In addition, 12-epi-scalaradial, which is one of the effective ingredients of the present invention, can be fractionated from a poriferan by a known method, or a fraction containing the effective ingredient in a high concentration can be also used as the effective ingredient of the present invention. The above-mentioned fractionation means includes extraction, separation by precipitation, column chromatography, thin layer chromatography, and the like. By further progressing purification of the resulting fraction using, as an index, an enhancing action on glucose uptake into a cell, or an action for inducing differentiation into an adipocyte, in other words, an insulin-mimetic action, as illustrated in Examples 1 and 5 set forth below, the effective ingredient can be also isolated.

No toxicity is especially found in the effective ingredient of the present invention as mentioned later. Also, there is no risk of the onset of side effects. For these reasons, the disease can be safely and appropriately treated or prevented. Therefore, the therapeutic agent, the prophylactic agent, the food, the beverage or the feed, each comprising the effective ingredient, is effective in treating or preventing a disease accompanying an abnormality in an amount of insulin or insulin response.

In the present invention, the disease accompanying an abnormality in an amount of insulin or insulin response includes diseases characterized by a factor selected from change in insulin level in blood, change in activity level of insulin or an insulin receptor, aberrance in downstream signal of an insulin receptor and a combination thereof. The disease is exemplified by, for example, diabetes, obesity, hypertension, arteriosclerosis, cocaine withdrawal symptoms, static cardiac incompetence, cardiovascular spasm, cerebral angiospasm, chromaffinoma, ganglioneuroblastoma, Huntington's disease, hyperlipemia and hyperinsulinemia. The diabetes may be exemplified by both of type I diabetes and type II diabetes. In addition, the type II diabetes encompasses a disease of which causation is insulin resistance for which a therapeutic effect is not found even when insulin or an agent for enhancing insulin secretion is administered.

In addition, the disease accompanying an abnormality in an amount of insulin or insulin response is more likely to lead to deficiency in the amount of insulin production, or deficiency in the action by insulin caused by insulin resistance in the onset stage of the disease. Since the effective ingredient of the present invention can suppress the onset of the disease accompanying an abnormality in an amount of insulin or insulin response by exhibiting an insulin-mimetic action, the prophylactic effects of the disease can be also expected.

In the state of insulin resistance, a signal from an insulin receptor by insulin is inhibited, so that diversified functions possessed by insulin are not exhibited, whereby generating various dysbolisms. The effective ingredient used in the present invention can exhibit an insulin-mimetic effect also for a symptom of insulin resistance, as described in Example 27. Specifically, by using the prophylactic agent or therapeutic agent of the present invention, a therapeutic or prophylactic effect can be exhibited also for a disease caused by insulin resistance, for example, type II diabetes for which a therapeutic effect is not seen even when insulin or an agent for enhancing insulin secretion is administered. In addition, the effective ingredient of the present invention can also exhibit the effect of lowering the amount of insulin in blood. In other words, the medicament of the present invention can be also used as a therapeutic agent or prophylactic agent for a disease requiring lowering of the amount of insulin for treatment or prevention. The disease is not particularly limited, and is exemplified by hyperinsulinemia, Alzheimer's disease and the like. In addition, since reports have been made that the stimulation via an insulin receptor and the effect of extended longevity are closely related *(Science,* vol. 299, P572-574 (2003); *Nature*, vol. 424, P277-284 (2003)), the medicament of the present invention can also be used as an agent for anti-aging.

There have been known that insulin enhances induction of differentiation of preadipocytes into adipocytes, and that glucose uptake takes place in matured adipocytes thereby accumulating triglyceride in the adipocytes *(J. Biol. Chem.,* Vol. 253, No. 20, P7570-7578 (1978)). Specifically, utilizing this method, an insulin-mimetic action of a test substance can be determined by administering the test substance in place of insulin, and determining differentiation into an adipocyte and the amount of triglyceride in the adipocytes.

In addition, there have been known that insulin has an enhancing action on glucose uptake, and that glucose uptake into a cell is enhanced by the action of insulin in a matured adipocyte *(J. Biol. Chem*., Vol. 253, No. 20, P7579-7583 (1978)). Specifically, utilizing this method, an insulin-mimetic action of a test substance can be determined by administering the test substance in place of insulin, and determining the amount of glucose uptake into a matured adipocyte.

The therapeutic agent or prophylactic agent of the present invention includes ones formed into a preparation by combining the above-mentioned effective ingredient according to the present invention with a known pharmaceutical carrier. In the therapeutic agent or prophylactic agent of the present invention, as the effective ingredient in the form of a salt, a pharmacologically acceptable salt is used. Also, as the therapeutic agent or prophylactic agent of the present invention, the above-mentioned effective ingredient can be combined with other components which can be used for the same applications as those of the effective ingredients, for example, insulin preparation, an agent for enhancing insulin secretion, an agent for improving insulin resistance, an agent for ameliorating postprandial hyperglycemia, an agent for insulin-mimetic action or the like which is known in the art. In addition, the therapeutic agent or prophylactic agent can be combined with a processed product derived from a plant belonging to Umbelliferae having an insulin-mimetic action described in WO 04/014407.

The therapeutic agent or prophylactic agent of the present invention is usually manufactured by combining the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like can be optionally added thereto, to form a solid agent such as a tablet, a granule, a powder, an epipastic, and a capsule, or a liquid agent such as a common liquid agent, a suspension agent or an emulsion agent. In addition, there can be also formed into a dry product which can be liquefied by adding an appropriate carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for example, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a corrective, a colorant, a flavor, and the like can be further combined therewith. For example, in the case of forming into a tablet or a pill, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for example, purified water, ethanol or the like is utilized as a carrier. Furthermore, an auxiliary agent such as a wetting agent or a suspending agent, a sweetening agent, a flavor, an antiseptic, or the like may be added as desired.

On the other hand, in the case of a parenterally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, a tonicity agent, a soothing agent, or the like if needed. It is also possible to produce a solid composition and dissolve the composition in sterile water or a sterile solvent for injection before use.

The external preparation includes solid, semi-solid or liquid preparations for percutaneous administration or transmucosal (intraoral or intranasal) administration. The external preparation also includes suppositories and the like. For example, the external preparation may be prepared as liquid preparations including emulsions, suspensions such as lotions, external tinctures, and liquid agents for transmucosal administration; ointments such as oily ointments and hydrophilic ointments; patches for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is in an amount so that the effective ingredient can be preferably administered within the dose range described below in consideration of administration form, administration method and the like of the preparation. The content of the effective ingredient in the medicament of the present invention is from 0.1 to 100% by weight or so.

The therapeutic agent or prophylactic agent of the present invention is administered via an administration route appropriate for each of the preparation form. The administration method is also not limited to specific one. The agent can be administered internally, externally (or topically) and by injection. The injection can be administered, for example, intravenously, intramuscularly, subcutaneously, intracutaneously, or the like. As to an external preparation, for example, a suppository may be administered according to its proper administration method.

The dose of the therapeutic agent or prophylactic agent of the present invention is changeable and properly set depending upon its preparation form, administration method, purpose of use, and age, weight, symptom or the like of a patient to which the therapeutic agent or prophylactic agent is administered, or the like. Generally, the dose of the agent, in terms of the dose of the above-mentioned effective ingredient contained in the preparation, is, for example, from 0.1 µg to 10 g/kg weight, preferably from 1 µg to 5 g/kg weight, and even more preferably from 10 µg to 1 g/kg weight, per day for adult. As a matter of course, the dose varies depending upon various conditions, so that an amount smaller than the dose mentioned above may be sufficient, or an amount exceeding the dose range may be required. Administration may be carried out once or in several divided portions in a day within the desired dose range. The administration period may be arbitrarily determined. Also, the therapeutic agent or prophylactic agent of the present invention can be directly orally administered, or the agent can be added to any foodstuffs to be taken on a daily basis.

In addition, the present invention can provide an agent for insulin-mimetic action comprising the above-mentioned effective ingredient. The agent for insulin-mimetic action may be the above-mentioned effective ingredient itself, or a composition comprising the above-mentioned effective ingredient. In the embodiment of the present invention, as the effective ingredient in the form of a salt, a pharmacologically acceptable salt is preferable. The agent for insulin-mimetic action may be prepared by, for example, combining the above-mentioned effective ingredient with other component, for example, insulin preparation, an agent for enhancing insulin secretion, an agent for improving insulin resistance, an agent for ameliorating postprandial hyperglycemia, an agent for insulin-mimetic action or the like which is known in the art which can be used for the same application as the effective ingredient, and forming into a form of reagent usually used according to the above-mentioned process for preparing the therapeutic agent or prophylactic agent. In addition, the agent for insulin-mimetic action can be combined with a processed product derived from a plant belonging to Umbelliferae having an insulin-mimetic action described in WO 04/014407. The content of the above-mentioned effective ingredient in the agent for insulin-mimetic action is not particularly limited, as long as the content is in an amount so that the desired effects of the present invention can be exhibited in consideration of administration method, purpose of use or the like of the agent for insulin-mimetic action. The content of the effective ingredient in the agent for insulin-mimetic action of the present invention is from 0.1 to 100% by weight or so. Also, the amount of the agent for insulin-mimetic action used is not particularly limited, as long as the desired effects of the present invention can be exhibited. Especially in the case where the agent for insulin-mimetic action is administered to a living body, the agent for insulin-mimetic action may be preferably used in an amount so that the effective ingredient can be administered within the dose range of the effective ingredient for the above-mentioned therapeutic agent or prophylactic agent. The agent for insulin-mimetic action is useful in treating or preventing a disease accompanying an abnormality in an amount of insulin or insulin response. In addition, the agent for insulin-mimetic action is useful in screening of drugs for diseases accompanying an abnormality in an amount of insulin or insulin response. Furthermore, the agent for insulin-mimetic action is useful in studies on mechanisms of an action on cells by insulin, or functional studies relating to physical changes in the cells. In addition, the agent for an insulin-mimetic action can be used by adding the agent in place of or together with serum or insulin preparation to a medium for culturing a cell, a tissue, or an organ. The medium is very useful as a medium for culturing a cell, a tissue or an organ that has reduced level of, or contains no serum or insulin preparation.

In addition, the amount of insulin in blood can be expected to be lowered by administering the agent for insulin-mimetic action of the present invention to human. In other words, the agent for insulin-mimetic action of the present invention can also be used as a therapeutic or prophylactic agent for a disease requiring the lowering of the amount of insulin for the treatment or prevention. The disease is not particularly limited, and is exemplified by hyperinsulinemia, Alzheimer's disease and the like. In addition, since reports have been made that the stimulation via an insulin receptor and the effect of extended longevity are closely related (*Science,* vol. 299, P572-574 (2003); *Nature*, vol. 424, P277-284 (2003)), the agent for insulin-mimetic action of the present invention can also be used as an agent for anti-aging.

No toxicity is especially found in the effective ingredient according to the present invention as described later. Also, there is no risk of the onset of side effects. For these reasons, the insulin-mimetic action can be safely and appropriately exhibited in a living body. Therefore, the medicament, food, beverage or feed of the present invention comprising the effective ingredient is effective in treating or preventing a disease accompanying an abnormality in an amount of insulin or insulin response.

In addition, the present invention provides a food, beverage or feed for treating or preventing a disease involved in a disease accompanying an abnormality in an amount of insulin or insulin response, wherein the food, beverage or feed comprises the above-mentioned effective ingredient. As the effective ingredient in the form of a salt used in the food, beverage or feed of the present invention, a pharmacologically acceptable salt or a salt of the same level of safety is preferable. Since the food, beverage or feed of the present invention has an insulin-mimetic action, the food, beverage or feed is very useful in amelioration of symptoms or prevention of a disease accompanying an abnormality in an amount of insulin or insulin response. Furthermore, the food or beverage of the present invention is a food or beverage for lowering blood glucose level, having the action of lowering a blood glucose level, so that the food or beverage is useful as a functional food or beverage effective in an individual who cares about his/her blood glucose level or an individual who cares about his/her body fat.

The food, beverage or feed of the present invention can be combined with another substance which is known to have an anti-diabetic action, for example, a substance having an insulin-mimetic action, a substance having enhancing action on insulin secretion, a substance having an action for improving insulin resistance, a substance having an action for ameliorating postprandial hyperglycemia or the like, which is commonly known. The food, beverage or feed can also be combined with, for example, hardly digestible dextrin or the like. In addition, the food, beverage or feed can be combined with a processed product derived from a plant belonging to Umbelliferae having an insulin-mimetic action described in WO 04/014407.

The term "comprising" in the food, beverage or feed of the present invention intended to mean containing(ed), adding(ed) and/or diluting(ed). As used herein, the term "containing(ed)" refers to an embodiment of containing the effective ingredient used in the present invention in the food, beverage or feed; the term "adding(ed)" refers to an embodiment of adding the effective ingredient used in the present invention to a raw material for the food, beverage or feed; and the term "diluting(ed)" refers to an embodiment of adding a raw material for the food, beverage or feed to the effective ingredient used in the present invention.

The process for preparing the food, beverage or feed of the present invention is not particularly limited. For example, combination, cooking, processing, and the like can be carried out in accordance with those generally employed for foods, beverages or feeds, and the food, beverage or feed of the present invention can be prepared by the methods for preparing a food, beverage or feed, as long as the resulting food, beverage or feed contains the above-mentioned effective ingredient of the present invention having insulin-mimetic action.

The food or beverage of the present invention is not particularly limited. The food or beverage includes, for example, processed agricultural and forest products, processed livestock products, processed marine products and the like, including processed grain products such as processed wheat products, processed starch products, processed premix products, noodles, macaronis, bread, bean jam, buckwheat noodles, wheat-gluten bread, rice noodles, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, steamed fish paste, tubular roll of steamed fish paste, cake of ground fish, deep-fried patty of fish paste, fish ball, steamed fish paste of fascia and tendon of fish *(suji),* fish meat ham, sausage, dried bonito, products of processed fish egg, canned marine products, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor (*shochu*), vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, bottled or pouched foods such as rice topped with cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like, in which each of the foods and beverages comprises the above-mentioned ingredient according to the present invention.

In the food or beverage of the present invention, the above-mentioned effective ingredient is contained, added and/or diluted, alone or in plurality, and its shape is not particularly limited, as long as the effective ingredient is contained in an amount necessary for exhibiting its insulin-mimetic action. For example, the shape includes those which can be taken orally such as tablets, granules and capsules.

The content of the above-mentioned effective ingredient in the food or beverage of the present invention is not particularly limited, and the content can be appropriately selected from the viewpoints of sensory aspect and exhibition of activity. The content of the effective ingredient is, for example, preferably 0.00001% by weight or more, more preferably from 0.0001 to 10% by weight, even more preferably from 0.0006 to 6% by weight, of the food. The content is, for example, preferably 0.00001% by weight or more, more preferably from 0.0001 to 10% by weight, even more preferably from 0.0006 to 6% by weight, of the beverage. Also, the food or beverage of the present invention may be taken so that the effective ingredient contained therein is taken in an amount of, for example, from 0.1 µg to 10 g/kg weight, preferably from 1 µg to 5 g/kg weight, and more preferably from 10 µg to 1 g/kg weight, per day for adult.

In addition, the present invention provides a feed for an organism having insulin-mimetic action, in which the feed comprises the above-mentioned effective ingredient, in other words, the effective ingredient is contained, added and/or diluted in the feed. In still another embodiment, the present invention also provides a method of feeding an organism, characterized by administering the above-mentioned effective ingredient to the organism. In still yet another embodiment, the present invention provides an organism-feeding agent characterized in that the organism-feeding agent comprises the above-mentioned effective ingredient.

In these inventions, the organisms are, for example, cultured or bred animals, pet animals, and the like. The cultured or bred animal is exemplified by livestock, laboratory animals, poultry, pisces, crustacea or shellfish. The feed is exemplified by a feed for sustenance of and/or amelioration in physical conditions. The organism-feeding agent is exemplified by immersion agents, feed additives, and beverage additives.

According to these inventions, the same effects can be expected to be exhibited as those of the above-mentioned therapeutic agent or prophylactic agent of the present invention, on the basis of the insulin-mimetic action of the above-mentioned effective ingredient used in the present invention, in the organism exemplified above to which these are applied. In other words, the above-mentioned feed or the like has a therapeutic or prophylactic effect on a disease accompanying an abnormality in an amount of insulin or insulin response in the organism.

The above-mentioned effective ingredient used in the present invention is usually administered in an amount of preferably from 0.01 to 2000 mg per day per 1 kg of the weight of a subject organism. The administration can be carried out, for example, by previously adding and mixing the effective ingredient in a raw material for an artificially formulated feed to be given to a subject organism, or mixing the effective ingredient with a powder raw material for an artificially formulated feed, and thereafter further adding and mixing the mixture with other raw materials. The content of the above-mentioned effective ingredient in the feed is not particularly limited. The content can be appropriately set in accordance with its purposes, and the content is preferably in a ratio of from 0.001 to 15% by weight. The content of the effective ingredient of the present invention in the organism-feeding agent may be adjusted to the same level as the feed.

The process for preparing the feed according to the present invention is not particularly limited, and its composition may be set in accordance with a general feed, as long as the above-mentioned effective ingredient according to the present invention having insulin-mimetic action may be contained in the feed prepared. The organism feeding agent can also be prepared in the same manner.

The organism to which the present invention can be applied is not limited. The cultured or bred animals include livestock such as *Equus, Bos, Porcus, Ovis, Capra, Camelus,* and *Lama*; laboratory animals such as mice, rats, guinea pigs, and rabbits; poultry such as *Chrysolophus,* ducks, *Meleagris,* and *Struthioniformes;* and the pet animals include dogs, cats, and the like, so that the present invention can be widely applied.

In the present invention, by, for example, allowing a subject organism to take the feed comprising the above-mentioned effective ingredient used in the present invention having insulin-mimetic action, or immersing a subject organism into a solution containing the above-mentioned effective ingredient used in the present invention having insulin-mimetic action (for example, prepared by dissolving the above immersing agent in water), the physical conditions of the livestock, laboratory animals, poultry, pet animals or the like can be well sustained or ameliorated. These embodiments are one embodiment of the feeding method of an organism in the present invention.

In addition, the present invention can provide an agent for enhancing glucose uptake into a cell comprising the above-mentioned effective ingredient. The agent for enhancing glucose uptake may be the above-mentioned effective ingredient itself, or a composition comprising the above-mentioned effective ingredient. As the effective ingredient in the form of a salt used in the agent for enhancing glucose uptake, a pharmacologically acceptable salt is used. The agent for enhancing glucose uptake may be prepared by, for example, combining the above-mentioned effective ingredient with other components, for example, insulin preparation, an agent for enhancing insulin secretion, an agent for improving insulin resistance, an agent for ameliorating postprandial hyperglycemia, an agent for insulin-mimetic action or the like that is commonly known, which can be used for the same application as the effective ingredient, and forming into a form of reagent usually used according to the above-mentioned process for preparing the therapeutic agent or prophylactic agent. In addition, the agent for enhancing glucose uptake can be combined with a processed product derived from a plant belonging to Umbelliferae having an enhancing action on glucose uptake into a cell described in WO 04/014407. The content of the above-mentioned effective ingredient in the agent for enhancing glucose uptake is not particularly limited, as long as the content is in an amount so that the desired effects of the present invention can be exhibited in consideration of administration method, purpose of use or the like of the agent for enhancing glucose uptake. The content of the effective ingredient in the agent for enhancing glucose uptake of the present invention is from 0.1 to 100% by weight or so. Also, the amount of the agent for enhancing glucose uptake used is not particularly limited, as long as the desired effects of the present invention can be exhibited. Especially in the case where the agent for enhancing glucose uptake is used by administering to a living body, the agent for enhancing glucose uptake may be preferably used in an amount so that the effective ingredient can be administered within the dose range of the effective ingredient for the above-mentioned therapeutic agent or prophylactic agent. The agent for enhancing glucose uptake is useful in treating or preventing a disease requiring an enhancing action on glucose uptake into a cell for the treatment or prevention. The disease is exemplified by, for example, the above-mentioned disease requiring an insulin-mimetic action, as well as cardiac diseases, especially cardiac infarction and post-ischemic injury of the heart, and the like. In addition, since the agent for enhancing glucose uptake enhances glucose uptake by a cell, when the action is exhibited in a muscle cell, an action for enhancing muscles or an action for recovery from fatigue can be induced. Also, the agent for enhancing glucose uptake can be used for the manufacture of a food, beverage or feed for treating or preventing these diseases. The food, beverage, or feed can be used according to the above-mentioned food, beverage or feed of the present invention. In addition, the agent for enhancing glucose uptake is also useful in screening of drugs for diseases requiring an enhancing action on glucose uptake into a cell for the treatment or prevention. Furthermore, the agent for enhancing glucose uptake is useful in studies on mechanisms of action on glucose uptake by the cell, or functional studies on physical changes in the cell and the like.

In addition, the present invention can provide an agent for inducing differentiation into an adipocyte comprising the above-mentioned effective ingredient. The precursor cell that can be induced to differentiate into adipocyte by the agent for inducing differentiation is not particularly limited, as long as the cell is capable of differentiating into adipocytes. The precursor cell includes, for example, preadipocyte, fibroblast, mesenchymal stem cell and the like. The agent for inducing differentiation may be the above-mentioned effective ingredient itself, or a composition comprising the above-mentioned effective ingredient. As the effective ingredient in the form of a salt used in the agent for inducing differentiation, a pharmacologically acceptable salt is used. The agent for inducing differentiation may be prepared by, for example, combining the above-mentioned effective ingredient with other component, for example, insulin preparation, an agent for enhancing insulin secretion, an agent for improving insulin resistance, an agent for ameliorating postprandial hyperglycemia, an agent for insulin-mimetic action or the like which is commonly known, which can be used for the same application as the effective ingredient, and forming into a form of reagent usually used according to the above-mentioned process for preparing the therapeutic agent or prophylactic agent. In addition, the agent for inducing differentiation can be combined with a processed product derived from a plant belonging to Umbelliferae having an action for inducing differentiation into an adipocyte described in WO 04/014407. The content of the above-mentioned effective ingredient in the agent for inducing differentiation is not particularly limited, as long as the content is in an amount so that the desired effects of the present invention can be exhibited in consideration of administration method, method of use or the like of the agent for inducing differentiation. The content of the effective ingredient in the agent for inducing differentiation of the present invention is from 0.1 to 100% by weight or so. Also, the amount of the agent for inducing differentiation used is not particularly limited, as long as the desired effects of the present invention can be exhibited. Especially in the case where the agent for inducing differentiation is administered to a living body, the agent for inducing differentiation may be preferably used in an amount so that the effective ingredient can be administered within the dose range of the effective ingredient for the above-mentioned therapeutic agent or prophylactic agent. The agent for inducing differentiation is useful in treating or preventing a disease requiring an action for inducing differentiation into an adipocyte for the treatment or prevention. The disease is exemplified by, for example, the above-mentioned disease requiring an insulin-mimetic action, as well as gout, fatty liver, cholecystolithiasis, menoxenia, infertility, and the like. The agent for inducing differentiation can be used for the manufacture of a food, beverage or feed for treating or preventing these diseases. The food, beverage, or feed can be used according to the above-mentioned food, beverage or feed of the present invention. In addition, the agent for inducing differentiation is also useful in screening of drugs for diseases requiring an agent for inducing differentiation into an adipocyte for the treatment or prevention. Furthermore, the agent for inducing differentiation is useful in studies on mechanisms of action for inducing differentiation into an adipocyte, or functional studies on physical changes thereof and the like.

In addition, the present invention also provides a novel compound 3,5,6,7,8,8a-hexahydro-5,5,8a-trimethyl-1,2-naphthalenedimethanol or a salt thereof. The compound can be prepared, for example, by referring to Example 6 set forth below.

No toxicity is found even when the above-mentioned effective ingredient used in the present invention is administered in an effective dose for the exhibition of its action. For example, in the case of oral administration, no cases of deaths are found even when polygodial, epipolygodial, isovelleral, 12-epi-scalaradial, naphthalene-2,3-dicarboxyaldehyde, miogadial, dimethyl 3,5,6,7,8,8a-hexahydro- 5,5,8a-trimethyl-1,2-naphthalenedicarboxylate, 3,5,6,7,8,8a-hexahydro-5,5,8a-trimethyl-1,2-naphthalenedimethanol, 4,6,7,8,9,9a-hexahydro-6,6,9a-trimethyl-naphtho[1,2-c]furan-1(3H)-one, dimethyl 1,4,4a,5,6,7,8,8a-octahydro-5,5,8a-trimethyl-1,2-naphthalenedicarboxylate, 1,4,4a,5,6,7,8,8a-octahydro- 5,5,8a-trimethyl-1,2-naphthalenedimethanol, 5,5a,6,7,8,9,9a,9b-octahydro- 6,6,9a-trimethylnaphtho[1,2-c]furan-3(1H)-one,1-[(acetyloxy)methyl]- 1,4,4a,5,6,7,8,8a-octahydro-5,5,8a-trimethyl-2-naphthalenecarboxyaldehyde, 2-[(acetyloxy)methyl]-1,4,4a,5,6,7,8,8a-octahydro-5,5,8a-trimethyl-1-naphthalenecarboxyaldehyde, or an optical isomer thereof or a salt thereof is administered to a mouse at 1g/kg weight in a single dose. In addition, no cases of deaths are found even when the above-mentioned effective ingredient is orally administered to a rat at 1 g/kg weight in a single dose.

### EXAMPLES

The present invention will be described more concretely hereinbelow by means of the examples, but the present invention is by no means limited to these descriptions. Unless specified otherwise, % in these examples means % by volume.

### Example 1 Enhancing Action by Polygodial on Glucose Uptake

### (1) Preparation of Mature Adipocytes

The induction of differentiation into mature adipocytes was carried out by partially modifying the method of Rubin C. S. et al. (*JBC*. **253** 7570-7578, 1978). 3T3-L1 cells (ATCC CCL-92.1) were suspended in a 10% calf serum (manufactured by ICN)-containing Dulbecco's modified Eagle's medium (manufactured by Sigma, D6046) containing 200 µM ascorbic acid so as to have a density of 4 × 10³ cells/mL, and the suspension was put in each well of a 12-well microtiter plate in an amount of 2 mL per well. The cells were cultured at 37°C for 7 days in the presence of 5% carbon dioxide gas. On the seventh day, the medium was exchanged with 2 mL of a 10% fetal bovine serum (manufactured by GIBCO)-containing Dulbecco's modified Eagle's medium containing 200 µM ascorbic acid, 0.25 µM dexamethasone, 10 µg/mL insulin (manufactured by TAKARA BIO INC.) and 0.5 mM 3-isobutyl-1-methylxanthine (manufactured by Nacalai Tesque, Inc., 19624-86). After 45 hours, the medium was exchanged with 2 mL of a 10% fetal bovine serum-containing Dulbecco's modified Eagle's medium containing 200 µM ascorbic acid and 5 µg/mL insulin. The medium was exchanged on after 2 days and 5 days, and the cells were cultured for 7 days, to give mature adipocytes.

### (2) Determination of Enhancing Action on Glucose Uptake into Mature Adipocytes

As the evaluation of enhancing action on glucose uptake, and also as the evaluation of insulin-mimetic action, the amount of 2-deoxyglucose uptake into mature adipocytes stimulated with the sample in the adipocytes was determined.

After the termination of the culture, the medium was removed, and the cells were washed twice with a 0.1% (w/v) bovine serum albumin (manufactured by Sigma, A8022)-containing Dulbecco's modified Eagle's medium. Thereafter, 1 mL of the same medium was put to each well. The cells were cultured overnight at 37°C in the presence of 5% carbon dioxide gas . After the overnight culture, the cells were washed twice with a HEPES buffered saline (140 mM NaCl, 5 mM KCl, 2.5 mM MgSO₄, 1 mM CaCl₂, 20 mM HEPES-Na (pH 7.4)), and 0.9 mL of the same buffer was added thereto. The cells were cultured at 37°C for 75 minutes. At the point when 45 minutes passed, as a sample, a dimethyl sulfoxide solution of polygodial (manufactured by Funakoshi Co., Ltd.) was added so as to have a final concentration of 3 µM, 1 µM, 0.3 µM, or 0.1 µM. During the culture, there were set a group with addition of insulin so as to have a final concentration of 1 µg/mL as a positive control, and a group without addition of the sample (group without addition; hereinafter referred to the same) as a negative control. Thereafter, 100 µL of a HEPES buffered saline containing 2-deoxy-[1,2-³H(N)]-glucose (manufactured by PerkinElmer Life Sciences Inc., NET549A) and 1 mM 2-deoxyglucose (manufactured by Nacalai Tesque, Inc., 10722-11) was added thereto at a final concentration of 0.5 µCi/mL, and the cells were further cultured at 37°C for 10 minutes. After the termination of the culture, the supernatant was removed, the cells were washed three times with a phosphate buffer cooled to 4°C, and 0.5 mL of a 1% Nonidet P-40-containing phosphate buffer was added to lyse the cells, whereby 2-deoxy-[1,2 ³H(N)]-glucose taken into the cells was eluted. The radioactivity was determined with a liquid scintillation counter LS6500 (manufactured by Beckmann) using 25 µL of the supernatant with Ultima Gold (manufactured by PerkinElmer Life Sciences Inc., 6013329) as a scintillation cocktail.

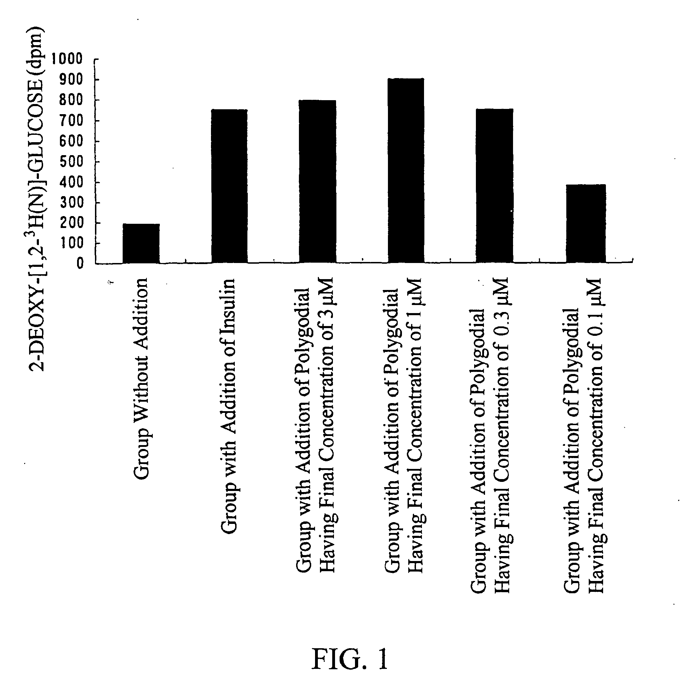

As a result, enhancement of 2-deoxy-[1,2-³H(N)]-glucose uptake was found in the group with addition of polygodial at each concentration as well as in the group with addition of insulin, as compared to the negative control. In other words, an enhancing action on glucose uptake was found in the polygodial. The results are shown in Figure 1. In Figure 1, the axis of abscissas is each sample, and the axis of ordinates is the amount of 2-deoxy-[1,2-³H(N)]-glucose (dpm).

### Example 2 Enhancing Action by 12-epi-scalaradial on Glucose Uptake

The amount of 2-deoxyglucose uptake into mature adipocytes stimulated with the sample in the adipocytes was determined in accordance with the method described in Example 1 as the evaluation of the enhancing action of 12-epi-scalaradial (manufactured by Funakoshi Co., Ltd., ST-350) on glucose uptake and also as the evaluation of the insulin-mimetic action.

Specifically, as a sample, there was set a group with addition of a dimethyl sulfoxide solution of 12- epi-scalaradial so as to have a final concentration of 10 µM, 3 µM or 1 µM. There were set a group without addition of the sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

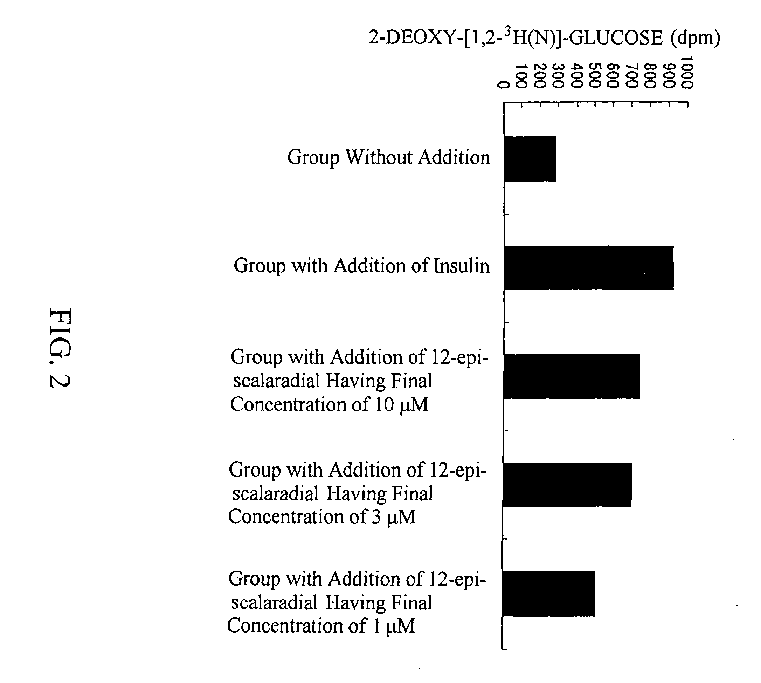

As a result, enhancement of 2-deoxy-[1,2-³H(N)]-glucose uptake was found in the group with addition of 12-epi-scalaradial as well as in the group with addition of insulin, as compared to the negative control. In other words, an enhancing action on glucose uptake was found in 12-epi-scalaradial. The results are shown in Figure 2. In Figure 2, the axis of abscissas is each sample, and the axis of ordinates is the amount of 2-deoxy-[1,2-³H(N)]-glucose (dpm).

### Example 3 Enhancing Action by Naphthalene-2,3-dicarboxyaldehyde on Glucose Uptake

The amount of 2-deoxyglucose uptake into mature adipocytes stimulated with the sample in the adipocytes was determined in accordance with the method described in Example 1 as the evaluation of enhancing action by naphthalene-2,3-dicarboxyaldehyde (manufactured by Funakoshi Co., Ltd.: N-1138) on glucose uptake, and also as the evaluation of insulin-mimetic action.

Specifically, as a sample, there was set a group with addition of a dimethyl sulfoxide solution of naphthalene-2,3-dicarboxyaldehyde so as to have a final concentration of 10 µM, 3 µM or 1 µM. There were set a group without addition of the sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

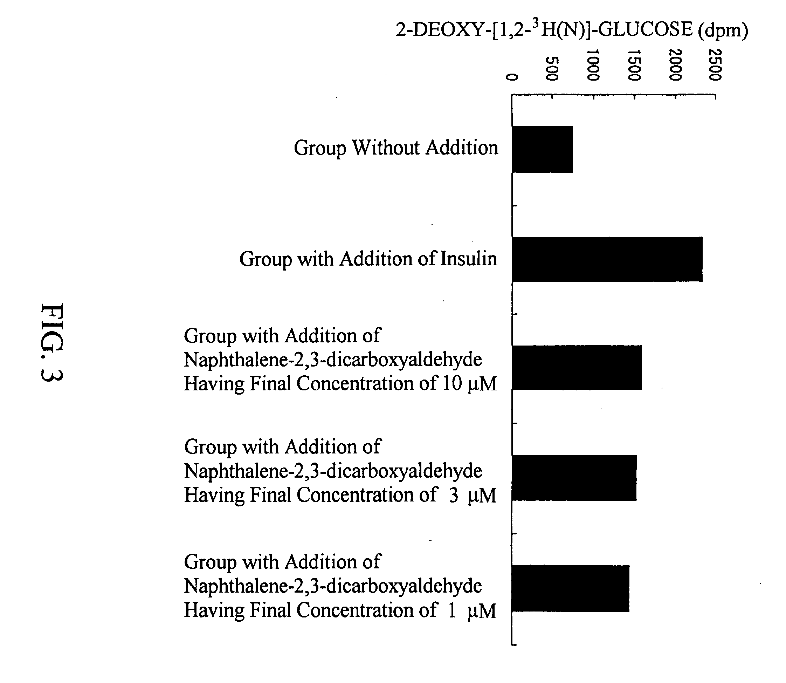

As a result, enhancement of 2-deoxy-[1,2-³H(N)]-glucose uptake was found in the group with addition of naphthalene-2,3- dicarboxyaldehyde as well as in the group with addition of insulin, as compared to the negative control. In other words, an enhancing action on glucose uptake was found in naphthalene-2,3-dicarboxyaldehyde. The results are shown in Figure 3. In Figure 3, the axis of abscissas is each sample, and the axis of ordinates is the amount of 2-deoxy-[1,2-³H(N)]-glucose (dpm).

### Example 4 Preparation of Compound (P001)

β-Cyclocitral (manufactured by Wako Pure Chemical Industries, Ltd.) was reacted in a hexane solution of 1 M trimethylsilylmethylmagnesium chloride (manufactured by Aldrich). Thereafter, the reaction mixture was treated with a catalytic amount of p-toluenesulfonic acid while refluxing with acetone. The resulting reaction product (1-vinyl-2,6,6-trimethyl-1-cyclohexene) was reacted with dimethyl acetylenedicarboxylate (manufactured by Wako Pure Chemical Industries, Ltd.) at 110°C for 20 hours, and the reaction mixture was subjected to silica chromatography to give a compound (P001).
¹H-NMR: δ1.15 (3H, s, -CH₃), 1.20 (3H, s, -CH₃), 1.44 (3H, s, -CH₃), 1.30-1.90 (6H, m, -(CH₂)₃-), 2.80 (1H, dd, J2.4, 23 Hz, =CH-CH₂-), 3.17 (1H, dd, J6.0, 23 Hz, =CH-CH₂-), 3.75 (3H, s, -OCH₃), 3.82 (3H, s, -OCH₃), 5.71 (1H, dd, J2.4, 6.0, =CH-CH₂-)

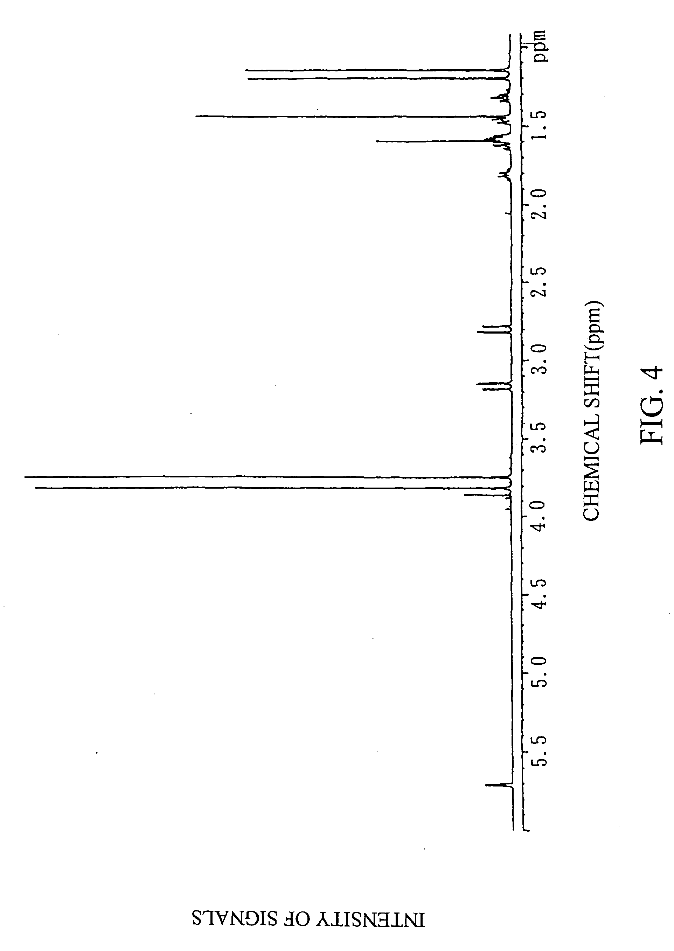

Here, the sample was dissolved in deuterated chloroform, and the chemical shift of the residual chloroform was expressed as 7.24 ppm. Figure 4 shows ¹H-NMR spectrum of the compound (P001). In Figure 4, the axis of abscissas is chemical shift (ppm), and the axis of ordinates is intensity of signal.

As the matrix, FAB-MS: m/z 293 (M+H)⁺ m-nitrobenzyl alcohol was used.

### Example 5 Inducing Action by Compound (P001) on Differentiation into Adipocytes

### (1) Induction of Differentiation into Adipocytes

Induction of differentiation into adipocytes was carried out by partially modifying the above-mentioned method of Rubin C. S. et al. In addition, as a compound (P001), a compound prepared in Example 4 was used. 3T3-L1 cells were suspended in a 10% (w/v) calf serum-containing Dulbecco's modified Eagle's medium containing 200 µM ascorbic acid so as to have a density of 4 × 10³ cells/mL, and the suspension was put in each well of a 12-well microtiter plate in an amount of 2 mL per well. The cells were cultured at 37°C for 7 days in the presence of 5% carbon dioxide gas. On the seventh day, the medium was exchanged with a 10% fetal bovine serum-containing Dulbecco's modified Eagle's medium containing 200 µM ascorbic acid and 0.25 µM dexamethasone, and 4 µL of a 15 mM dimethyl sulfoxide solution of the compound (P001), a 5 mM dimethyl sulfoxide solution of the compound (P001), or a 1.5 mM dimethyl sulfoxide solution of the compound (P001) was added to each well. Here, there were set a group with addition of 4 µL of a 5 mg/mL aqueous solution of insulin as a positive control, and a group with addition of dimethyl sulfoxide as a negative control. After 48 hours, the medium was exchanged with a 10% fetal bovine serum-containing Dulbecco's modified Eagle's medium containing 200 µM ascorbic acid, and 4 µL of a 15 mM dimethyl sulfoxide solution of the compound (P001), a 5 mM dimethyl sulfoxide solution of the compound (P001), or a 1.5 mM dimethyl sulfoxide solution of the compound (P001), a group with addition of 2 µL of a 5 mg/mL aqueous solution of insulin as a positive control, or a group with addition of dimethyl sulfoxide as a negative control was put to each well. The cells were cultured for additional 7 days. Here, the medium was exchanged after 2 days and after 5 days, and at that time, 4 µL of a 15 mM dimethyl sulfoxide solution of the compound (P001), a 5 mM dimethyl sulfoxide solution of the compound (P001), or a 1.5 mM dimethyl sulfoxide solution of the compound (P001), a group with addition of 2 µL of a 5 mg/mL aqueous solution of insulin as a positive control, or a group with addition of dimethyl sulfoxide as a negative control was put to each well. Here, the final concentrations of the compound (P001) in each medium are 30 µM, 10 µM, and 3 µM, respectively.

### (2) Determination of Amount of Biosynthesis of Triglyceride

The amount of triglyceride in the adipocytes was determined as an index for inducing differentiation into mature adipocytes, and also as the evaluation of insulin-mimetic action.

After the termination of the culture, the medium was removed, and the cells were washed twice with a phosphate buffered saline. One milliliter of a solvent of hexane: isopropanol = 3:2 was added to the cells. The mixture was allowed to stand at room temperature for 30 minutes, and thereafter the supernatant was collected. The procedures were repeated again, and 2 mL of the supernatant was concentrated to dryness. The precipitates were dissolved in 100 µL of isopropanol, and the amount of triglyceride contained in 10 µL of the solution was determined using a triglyceride E-test (manufactured by Wako Pure Chemical Industries, Ltd., code 432-40201). In addition, all the determinations were carried out twice.

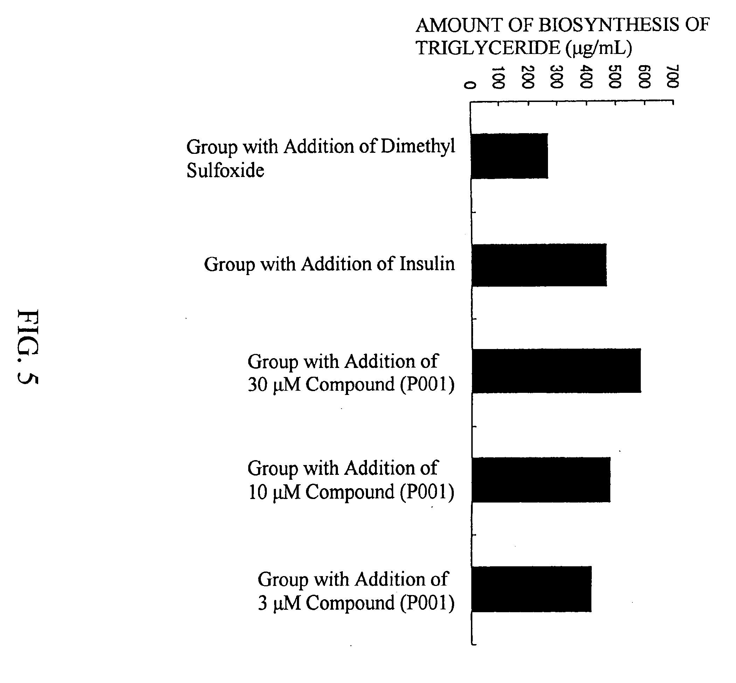

As a result, induction of biosynthesis of triglyceride was found in the group with addition of the compound (P001) at each concentration. In other words, an inducing action on differentiation into mature adipocytes was found in the compound (P001). The results are shown in Figure 5. In Figure 5, the axis of abscissas is each sample, and the axis of ordinates is the amount of biosynthesis of triglyceride (µg/mL).

### Example 6 Preparation of Compound (P002)

The compound (P001) was reacted with lithium aluminum hydride (manufactured by Wako Pure Chemical Industries, Ltd.) in ether at room temperature for 1 hour under an argon gas stream. The resulting reaction mixture was subjected to silica chromatography, thereby giving a compound (P002).
¹H-NMR: δ1.14 (3H, s, -CH₃), 1.19 (3H, s, -CH₃), 1.21 (3H, s, -CH₃), 1.20-2.00 (6H, m, -(CH₂)₃-), 2.85 (2H, d, J3.6 Hz, =CH-CH₂-), 4.13 (1H, d, J12 Hz, -CH₂OH), 4.21 (1H, d, J12 Hz, -CH₂OH), 4.27 (1H, d, J12 Hz, -CH₂OH), 4.35(1H, d, J12 Hz, -CH₂OH), 5.68 (1H, t, J3.6, =CH-CH₂-)

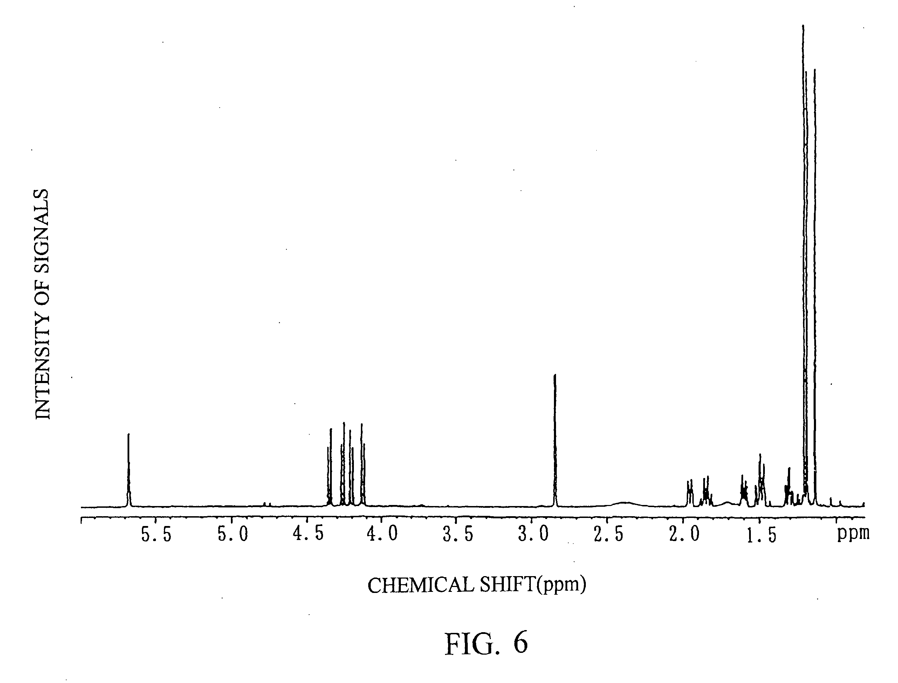

Here, the sample was dissolved in deuterated chloroform, and the chemical shift of the residual chloroform was expressed as 7.24 ppm. Figure 6 shows ¹H-NMR spectrum of the compound (P002). In Figure 6, the axis of abscissas is chemical shift (ppm), and the axis of ordinates is intensity of signal.

As the matrix, FAB-MS: m/z 237 (M+H)⁺ m-nitrobenzyl alcohol was used.

### Example 7 Inducing Action by Compound (P002) on Differentiation into Adipocytes

The inducing action on differentiation into mature adipocytes of the compound (P002) prepared in Example 6 (insulin-mimetic action) was determined in accordance with the method of Example 5. Specifically, as a sample, there was set a group with addition of a dimethyl sulfoxide solution of the compound (P002) so as to have a final concentration of 30 µM or 3 µM respectively to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

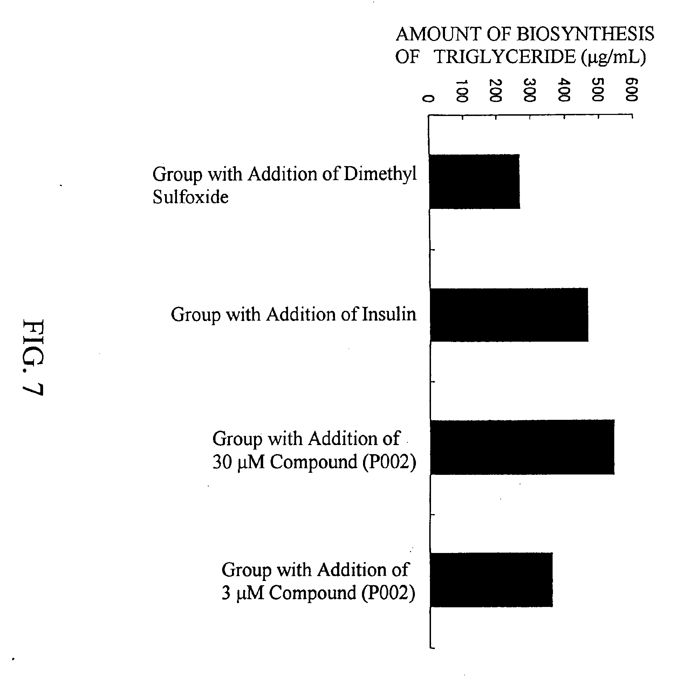

As a result, induction of biosynthesis of triglyceride was found in the group with addition of the compound (P002). In other words, an inducing action on differentiation into mature adipocytes was found in the compound (P002). The results are shown in Figure 7. In Figure 7, the axis of abscissas is each sample, and the axis of ordinates is the amount of biosynthesis of triglyceride (µg/mL).

### Example 8 Preparation of Compound (P003)

The compound (P002) was reacted with 15 molar equivalents of barium permanganate (manufactured by Wako Pure Chemical Industries, Ltd.) in dichloromethane at room temperature for 24 hours. The resulting reaction mixture was subjected to silica chromatography, thereby giving a compound (P003).
¹H-NMR: δ1.18 (3H, s, -CH₃), 1.24 (3H, s, -CH₃), 1.40 (3H, s, -CH₃), 1.30-2.00 (6H, m, -(CH₂)₃-), 2.82 (1H, m, =CH-CH₂-), 2.95 (1H, m, =CH-CH₂-), 4.74 (1H, m, -CO-O-CH₂-), 4.86 (1H, m, -CO-O-CH₂-), 5.77 (1H, m, =CH-CH₂-)

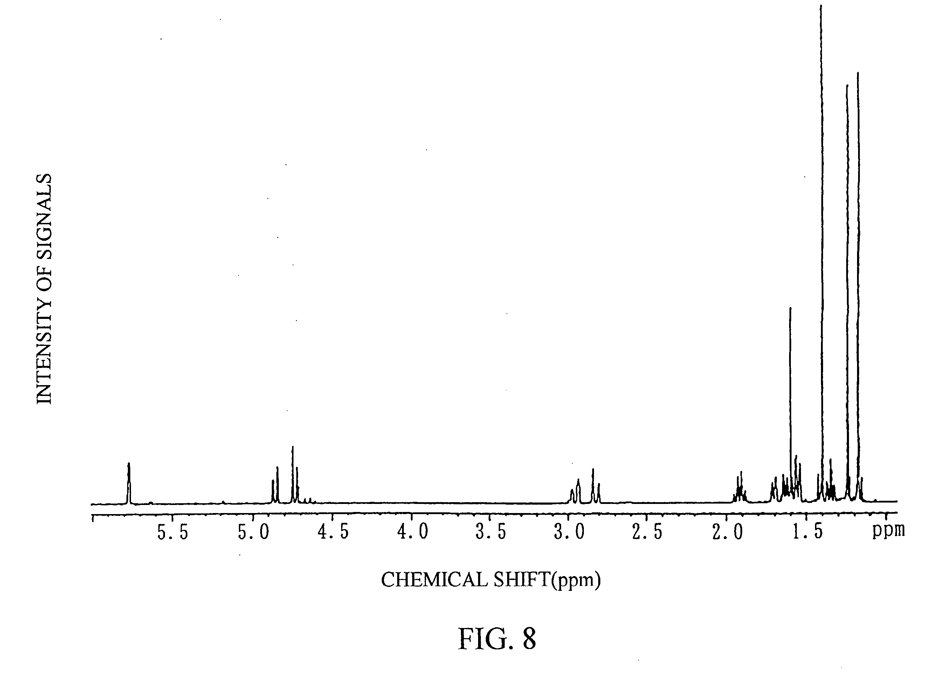

Here, the sample was dissolved in deuterated chloroform, and the chemical shift of the residual chloroform was expressed as 7.24 ppm. Figure 8 shows ¹H-NMR spectrum of the compound (P003). In Figure 8, the axis of abscissas is chemical shift (ppm), and the axis of ordinates is intensity of signal.

As the matrix, FAB-MS: m/z 233 (M+H)⁺ m-nitrobenzyl alcohol was used.

### Example 9 Inducing Action by Compound (P003) on Differentiation into Adipocytes

The inducing action on differentiation into mature adipocytes of the compound (P003) prepared in Example 8 (insulin-mimetic action) was determined in accordance with the method of Example 5. Specifically, there was set a group with addition of a dimethyl sulfoxide solution of the compound (P003) so as to have a final concentration of 30 µM, 10 µM or 3 µM as a sample respectively to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

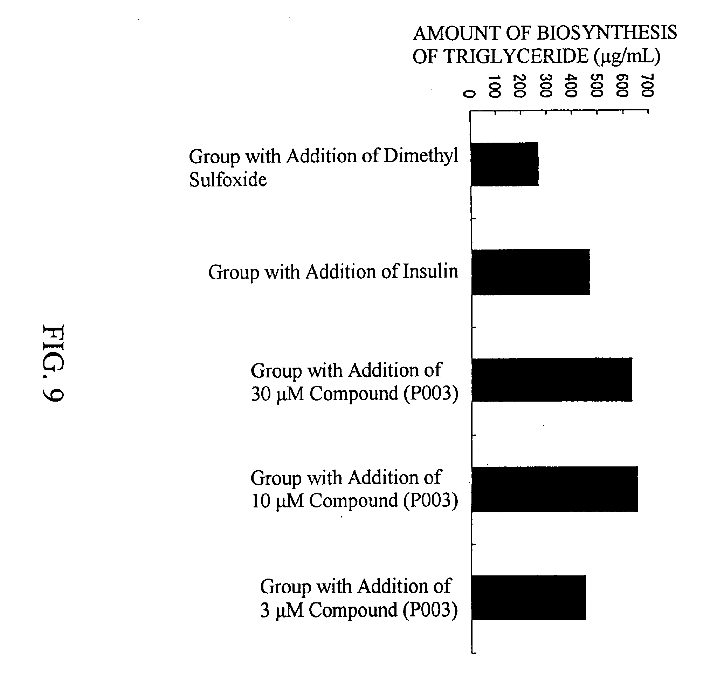

As a result, induction of biosynthesis of triglyceride was found in the group with addition of the compound (P003). In other words, an inducing action on differentiation into mature adipocytes was found in the compound (P003). The results are shown in Figure 9. In Figure 9, the axis of abscissas is each sample, and the axis of ordinates is the amount of biosynthesis of triglyceride (µg/mL).

### Example 10 Preparation of Compound (P004)

The compound (P001) was hydrogenated in the presence of a catalytic amount of palladium-active carbon (manufactured by Wako Pure Chemical Industries, Ltd.) in methanol. The resulting reaction mixture was subjected to silica chromatography, thereby giving a compound (P004).
¹H-NMR: δ0.89 (3H, s, -CH₃), 0.91 (3H, s, -CH₃), 0.95 (3H, s, -CH₃), 1.20-2.00 (7H, m), 2.13 (1H, m, -CH₂-CH=), 2.28 (1H, m, -CH₂-CH=), 3.20 (1H, m, -CH(COOCH₃)-), 3.68 (3H, s, -OCH₃), 3.71(3H, s, -OCH₃), 7.06 (1H, m, -CH₂-CH=)

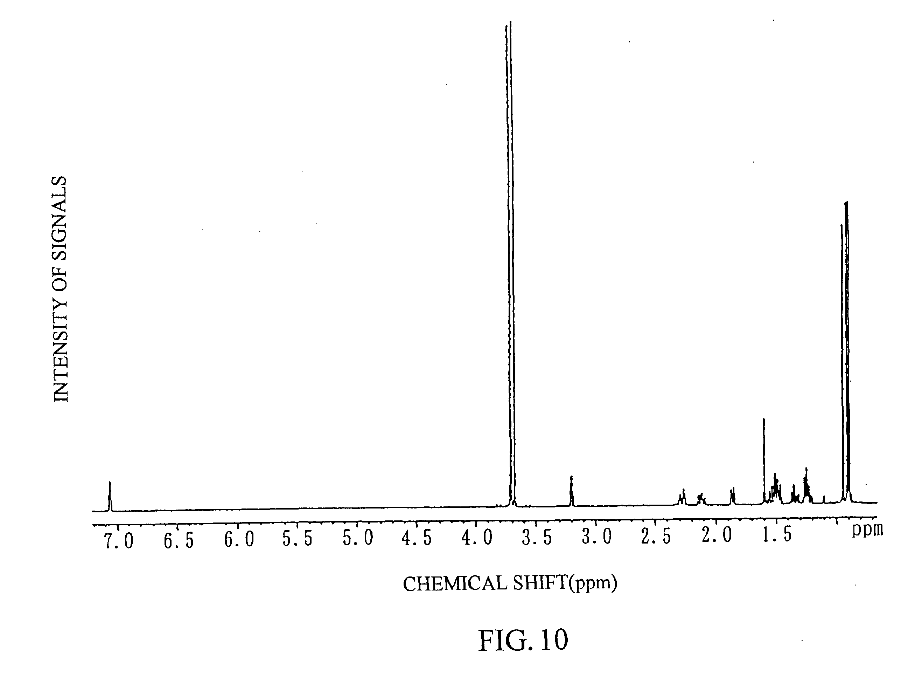

Here, the sample was dissolved in deuterated chloroform, and the chemical shift of the residual chloroform was expressed as 7.24 ppm. Figure 10 shows ¹H-NMR spectrum of the compound (P004). In Figure 10, the axis of abscissas is chemical shift (ppm), and the axis of ordinates is intensity of signal.

As the matrix, FAB-MS: m/z 295 (M+H)⁺ m-nitrobenzyl alcohol was used.

### Example 11 Enhancing Action by Compound (P004) on Glucose Uptake

The amount of 2-deoxyglucose uptake into mature adipocytes stimulated with the sample in the adipocytes was determined in accordance with the method described in Example 1 as the evaluation of enhancing action on glucose uptake and also as the evaluation of insulin-mimetic action of the compound (P004) prepared in Example 10.

Specifically, as a sample, there was set a group with addition of a dimethyl sulfoxide solution of the compound (P004) so as to have a final concentration of 30 µM, 10 µM, or 3 µM. Here, there were set a group without addition of a sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

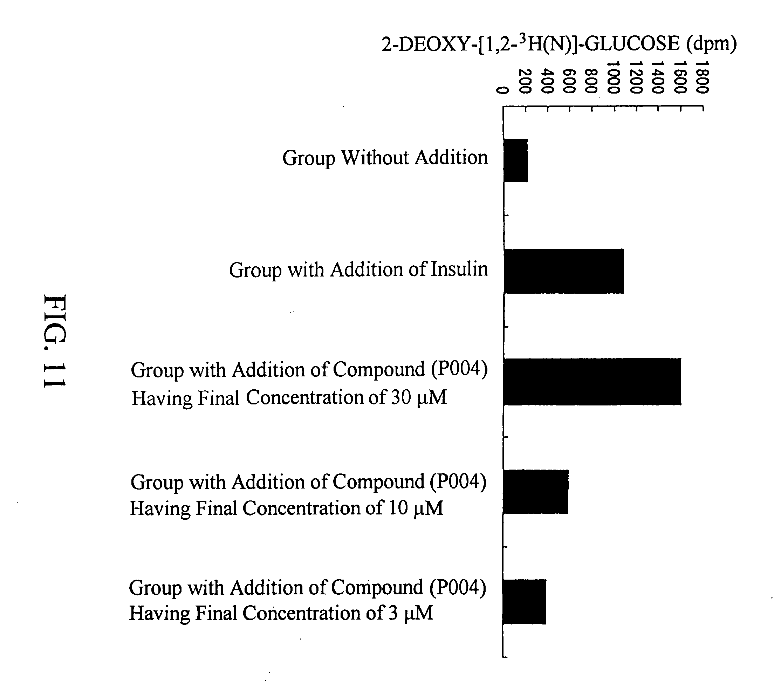

As a result, enhancement of 2-deoxy-[1,2-³H(N)]-glucose uptake was found in the group with addition of the compound (P004) as well as in the group with addition of insulin, as compared to the negative control. In other words, an enhancing action on glucose uptake was found in the compound (P004). The results are shown in Figure 11. In Figure 11, the axis of abscissas is each sample, and the axis of ordinates is the amount of 2-deoxy-[1,2-³H(N)]-glucose (dpm).

### Example 12 Inducing Action by Compound (P004) on Differentiation into Adipocytes

The inducing action on differentiation into mature adipocytes of the compound (P004) prepared in Example 10 (insulin-mimetic action) was determined in accordance with the method of Example 5. Specifically, as a sample, there was set a group with addition of a dimethyl sulfoxide solution of the compound (P004) so as to have a final concentration of 10 µM, 3 µM, or 1 µM, respectively to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

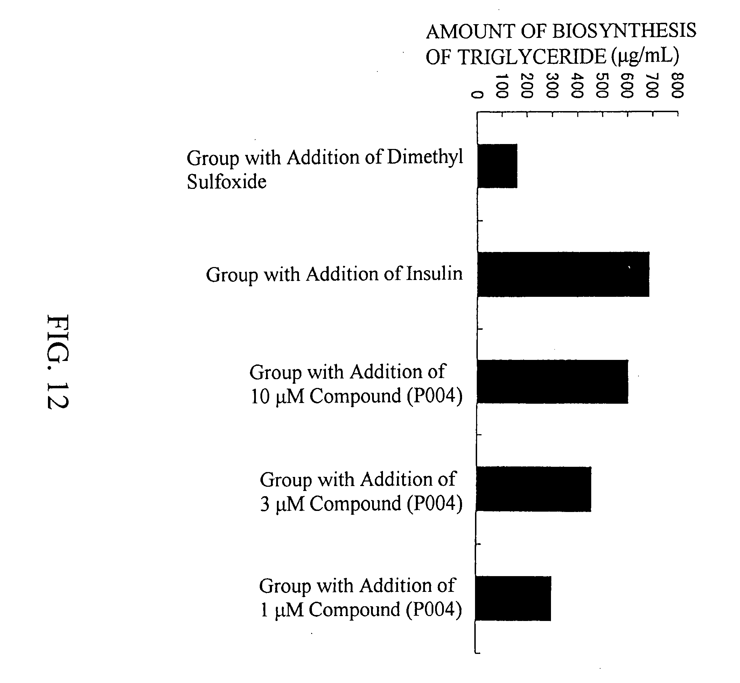

As a result, induction of biosynthesis of triglyceride was found in the group with addition of the compound (P004). In other words, an inducing action on differentiation into mature adipocytes was found in the compound (P004). The results are shown in Figure 12. In Figure 12, the axis of abscissas is each sample, and the axis of ordinates is the amount of biosynthesis of triglyceride (µg/mL).

### Example 13 Preparation of Compound (P005)

The compound (P004) was reacted with lithium aluminum hydride (manufactured by Wako Pure Chemical Industries, Ltd.) in ether at room temperature for 1 hour under an argon gas stream. The resulting reaction mixture was subjected to silica chromatography, thereby giving a compound (P005).
¹H-NMR: δ0.78 (3H, s, -CH₃), 0.89 (3H, s, -CH₃), 0.90 (3H, s, -CH₃), 1.10-2.00 (7H, m), 1.91 (1H, m, -CH₂-CH=), 2.11 (1H, m, -CH₂-CH=), 2.16 (1H, m, -CH(CH₂OH)-), 3.71 (1H, dd, J8.4, 11 Hz, -CH₂OH), 3.93 (1H, dd, J2.4, 11 Hz, -CH₂OH), 4.01 (1H, d, J12 Hz, -CH₂OH), 4.37 (1H, m, -CH₂OH), 5.82 (1H, m, -CH₂-CH=)

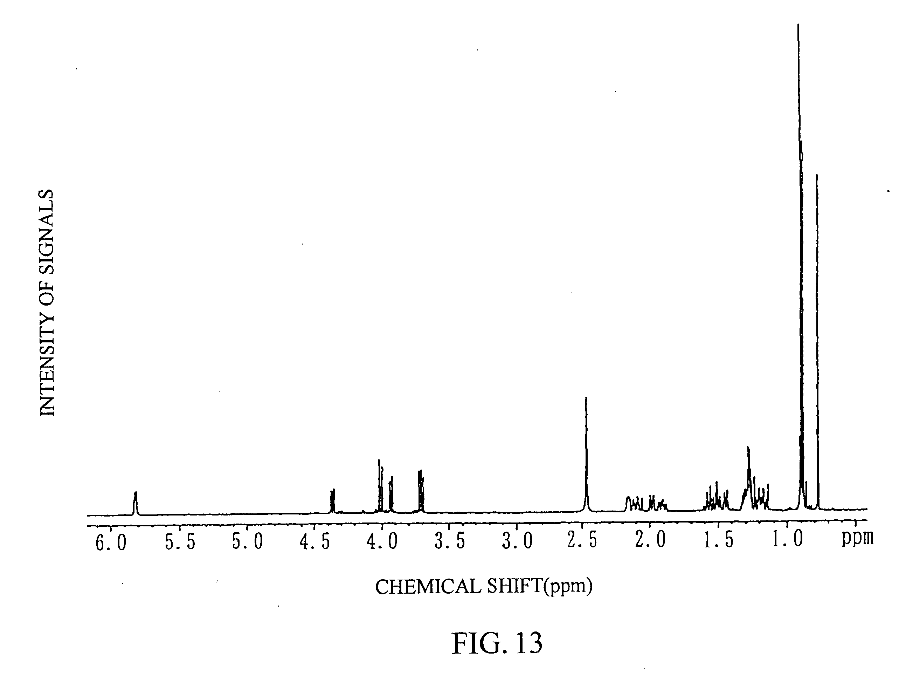

Here, the sample was dissolved in deuterated chloroform, and the chemical shift of the residual chloroform was expressed as 7.24 ppm. Figure 13 shows ¹H-NMR spectrum of the compound (P005). In Figure 13, the axis of abscissas is chemical shift (ppm), and the axis of ordinates is intensity of signal.

As the matrix, FAB-MS: m/z 239 (M+H)⁺ metanitrobenzyl alcohol was used.

### Example 14 Inducing Action by Compound (P005) on Differentiation into Adipocytes

The inducing action on differentiation into mature adipocytes of the compound (P005) prepared in Example 13 (insulin-mimetic action) was determined in accordance with the method of Example 5. Specifically, as a sample, there was set a group with addition of a dimethyl sulfoxide solution of the compound (P005) so as to have a final concentration of 30 µM or 10 µM respectively to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

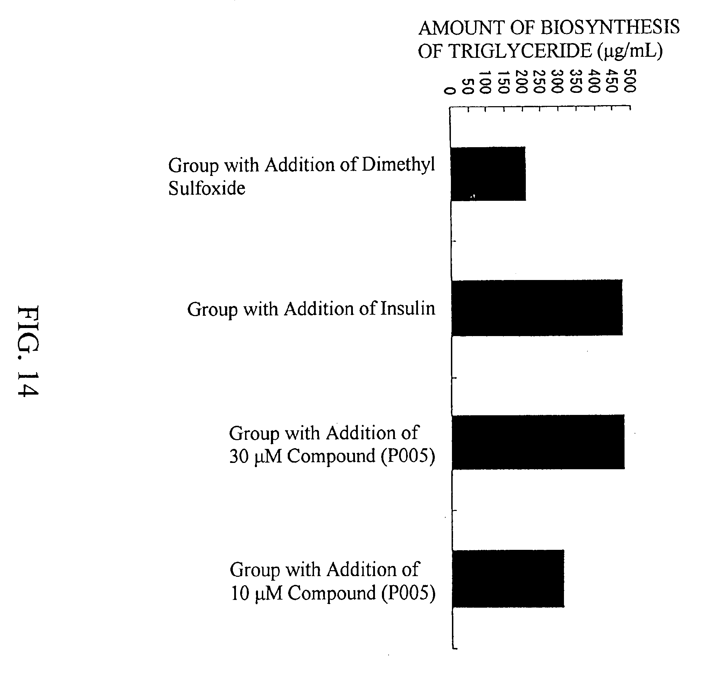

As a result, induction of biosynthesis of triglyceride was found in the group with addition of the compound (P005). In other words, an inducing action on differentiation into mature adipocytes was found in the compound (P005). The results are shown in Figure 14. In Figure 14, the axis of abscissas is each sample, and the axis of ordinates is the amount of biosynthesis of triglyceride (µg/mL).

### Example 15 Preparation of Compound (P006)

The compound (P005) was reacted with 15 molar equivalents of barium permanganate (manufactured by Wako Pure Chemical Industries, Ltd.) in dichloromethane at room temperature for 24 hours. The resulting reaction mixture was subjected to silica chromatography, thereby giving a compound (P006).
¹H-NMR: δ0.83 (3H, s, -CH₃), 0.94 (3H, s, -CH₃), 0.96 (3H, s, -CH₃), 1.20-1.70 (7H, m), 2.13 (1H, m,-CH₂-CH=), 2.43 (1H, m, -CH₂-CH=), 2.84 (1H, m, -CH-CH₂-O-), 4.05 (1H, t, J3.0 Hz, -CH-CH₂-O-), 4.39 (1H, t, J3.0 Hz, -CH-CH₂-O-), 6.89 (1H, m, -CH₂-CH=)

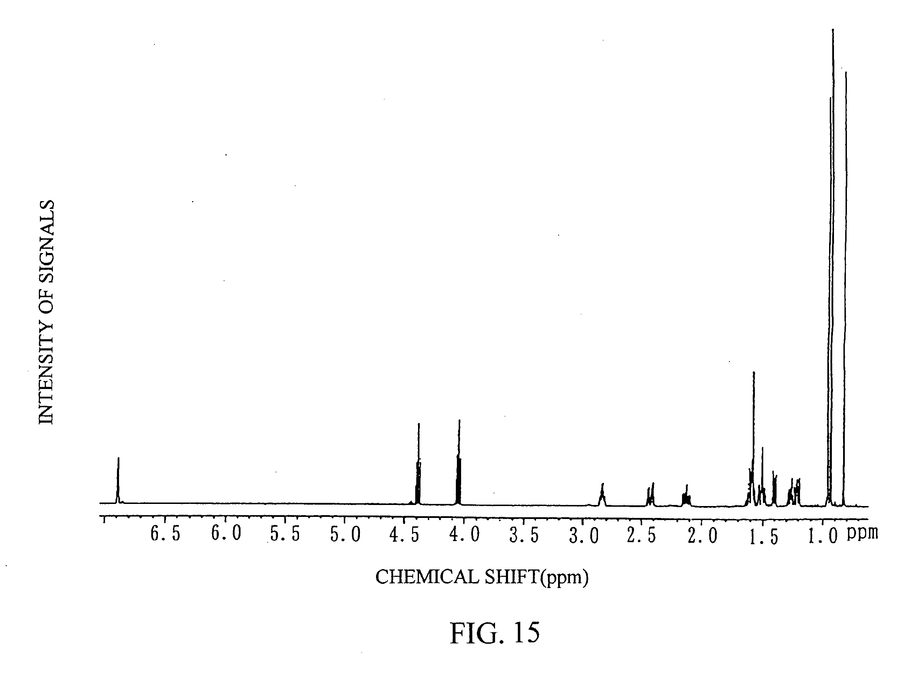

Here, the sample was dissolved in deuterated chloroform, and the chemical shift of the residual chloroform was expressed as 7.24 ppm. Figure 15 shows ¹H-NMR spectrum of the compound (P006). In Figure 15, the axis of abscissas is chemical shift (ppm), and the axis of ordinates is intensity of signal.

As the matrix, FAB-MS: m/z 235 (M+H)⁺ m-nitrobenzyl alcohol was used.

### Example 16 Inducing Action on Differentiation into Adipocytes by

### Compound (P006)

The inducing action on differentiation into mature adipocytes of the compound (P006) prepared in Example 15 (insulin-mimetic action) was determined in accordance with the method of Example 5. Specifically, as a sample, there was set a group with addition of a dimethyl sulfoxide solution of the compound (P006) so as to have a final concentration of 30 µM, 10 µM, or 3 µM respectively to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

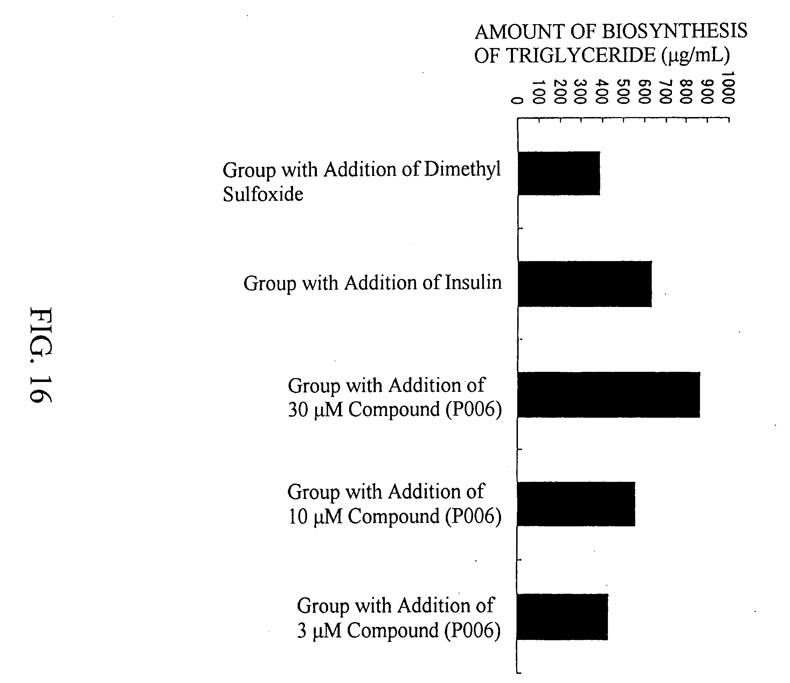

As a result, induction of biosynthesis of triglyceride was found in the group with addition of the compound (P006). In other words, an inducing action on differentiation into mature adipocytes was found in the compound (P006). The results are shown in Figure 16. In Figure 16, the axis of abscissas is each sample, and the axis of ordinates is the amount of biosynthesis of triglyceride (µg/mL).

### Example 17 Preparation of Compound (P007)

The compound (P005) was treated with t-butyldimethylchlorosilane in dichloromethane in the presence of triethylamine. The reaction mixture was treated with acetic anhydride in the presence of triethylamine, and thereafter treated with p-toluenesulfonic acid in methanol. The reaction mixture was purified by silica chromatography, thereby giving 1-[(acetyloxy)methyl]-1,4,4a,5,6,7,8,8a-octahydro-5,5,8a-trimethyl-2-naphthalenemethanol, and 2-[(acetyloxy)methyl]-1,4,4a,5,6,7,8,8a-octahydro-5,5,8a-trimethyl-1-naphthalenemethanol. The resulting 1-[(acetyloxy)methyl]-1,4,4a,5,6,7,8,8a-octahydro-5,5,8a-trimethyl-1-naphthalenemethanol was treated with manganese dioxide in dichloromethane, thereby giving a compound (P007).
¹H-NMR: δ0.90 (3H, s, -CH₃), 0.92 (3H, s, -CH₃), 0.97 (3H, s, -CH₃), 1.00-2.00 (7H, m), 1.96 (3H, s, -CO-CH₃), 2.25 (1H, m, -CH₂-CH=), 2.46 (2H, m, -CH₂-CH=, -CH-CHO), 4.43 (1H, dd, J2, 12 Hz, -CH₂-O-Ac), 4.63 (1H, dd, J6, 12 Hz, -CH₂-O-Ac), 6.94 (1H, m, -CH₂-CH=), 9.44 (1H, s, -CHO)

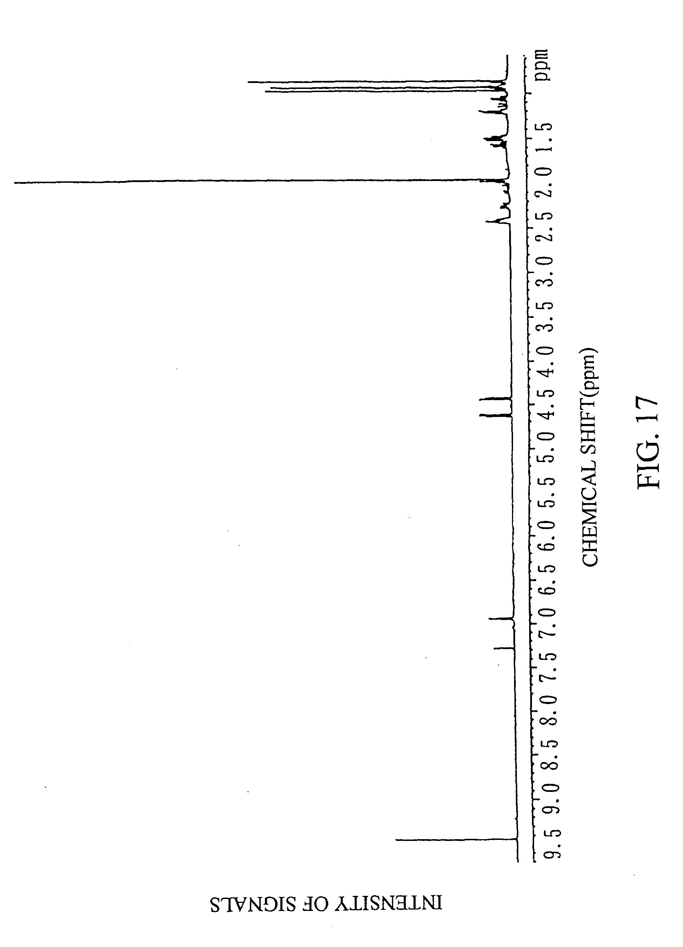

Here, the sample was dissolved in deuterated chloroform, and the chemical shift of the residual chloroform was expressed as 7.24 ppm. Figure 17 shows ¹H-NMR spectrum of the compound (P007). In Figure 17, the axis of abscissas is chemical shift (ppm), and the axis of ordinates is intensity of signal.

As the matrix, FAB-MS: m/z 279 (M+H)⁺ metanitrobenzyl alcohol was used.

### Example 18 Inducing Action by Compound (P007) on Differentiation into Adipocytes

The inducing action on differentiation into mature adipocytes of the compound (P007) prepared in Example 17 (insulin-mimetic action) was determined in accordance with the method of Example 5. Specifically, as a sample, there was set a group with addition of a dimethyl sulfoxide solution of the compound (P007) so as to have a final concentration of 30 µM, 10 µM, or 3 µM respectively, to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

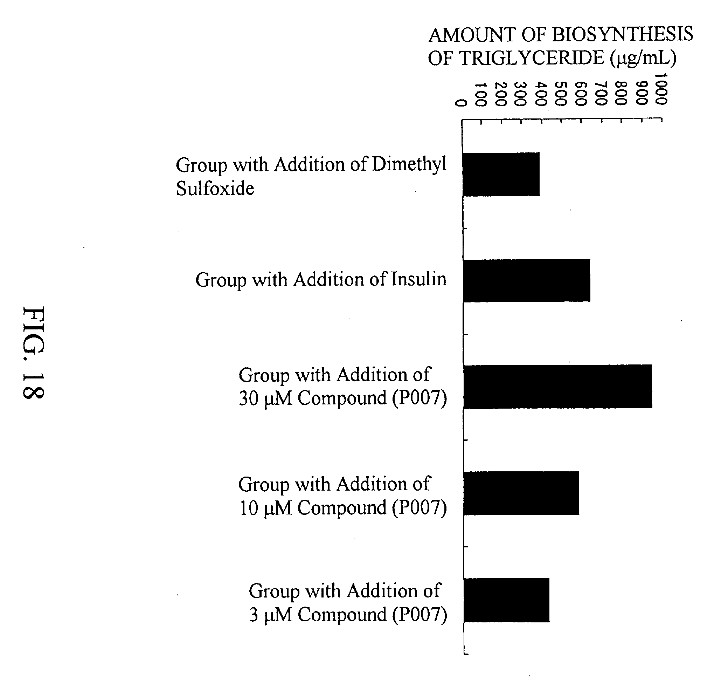

As a result, induction of biosynthesis of triglyceride was found in the group with addition of the compound (P007). In other words, an inducing action on differentiation into mature adipocytes was found in the compound (P007). The results are shown in Figure 18. In Figure 18, the axis of abscissas is each sample, and the axis of ordinates is the amount of biosynthesis of triglyceride (µg/mL).

### Example 19 Preparation of Compound (P008)

The resulting 2-[(acetyloxy)methyl]-1,4,4a,5,6,7,8,8a-octahydro-5,5,8a-trimethyl-1-naphthalenemethanol was treated with pyridium chlorochromate in dichloromethane in accordance with Example 17, thereby giving a compound (P008).
¹H-NMR: δ0.90 (3H, s, -CH₃), 0.94 (3H, s, -CH₃), 1.06 (3H, s, -CH₃), 1.20-1.80 (7H, m), 2.03 (3H, s, -CO-CH₃), 2.03 (1H, m, -CH₂-CH=), 2.19 (1H, m, -CH₂-CH=), 2.86 (1H, m, -CH-CHO), 4.44 (1H, d, J12 Hz, -CH₂-O-Ac), 4.57 (1H, m, -CH₂-O-Ac), 6.03 (1H, m, -CH₂-CH=), 9.47 (1H, d, J4.8 Hz, -CHO)

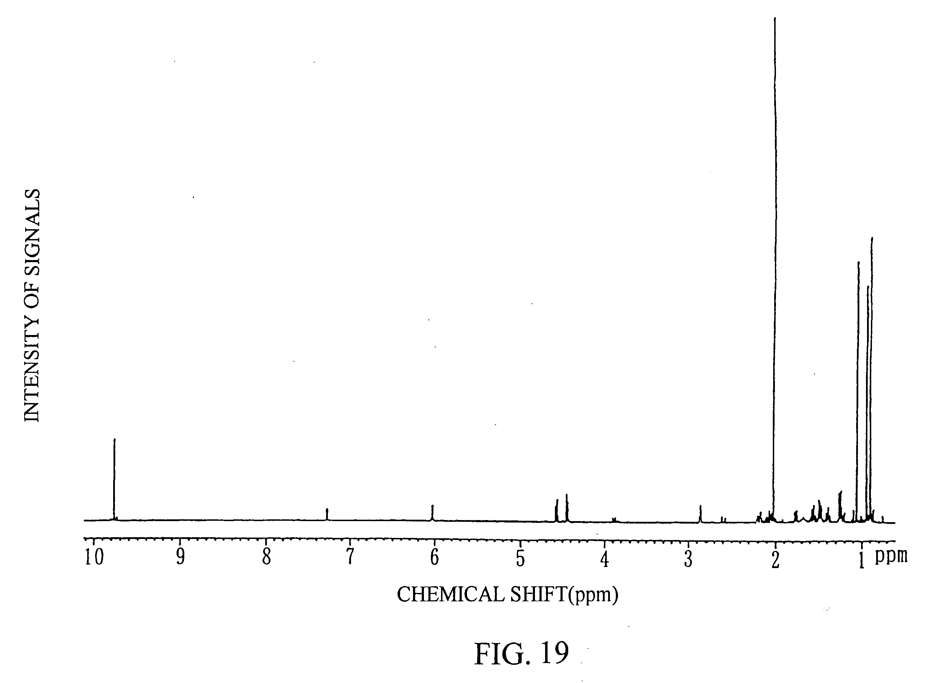

Here, the sample was dissolved in deuterated chloroform, and the chemical shift of the residual chloroform was expressed as 7.24 ppm. Figure 19 shows ¹H-NMR spectrum of the compound (P008). In Figure 19, the axis of abscissas is chemical shift (ppm), and the axis of ordinates is intensity of signal.

As the matrix, FAB-MS: m/z 279 (M+H)⁺ metanitrobenzyl alcohol was used.

### Example 20 Inducing Action by Compound (P008) on Differentiation into Adipocytes

The inducing action on differentiation into mature adipocytes of the compound (P008) prepared in Example 19 (insulin-mimetic action) was determined in accordance with the method of Example 5. Specifically, as a sample, there was set a group with addition of a dimethyl sulfoxide solution of the compound (P008) so as to have a final concentration of 10 µM or 3 µM respectively to each well. Here, there were set a group with addition of 4 µL of a 5 mg/mL aqueous solution of insulin as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

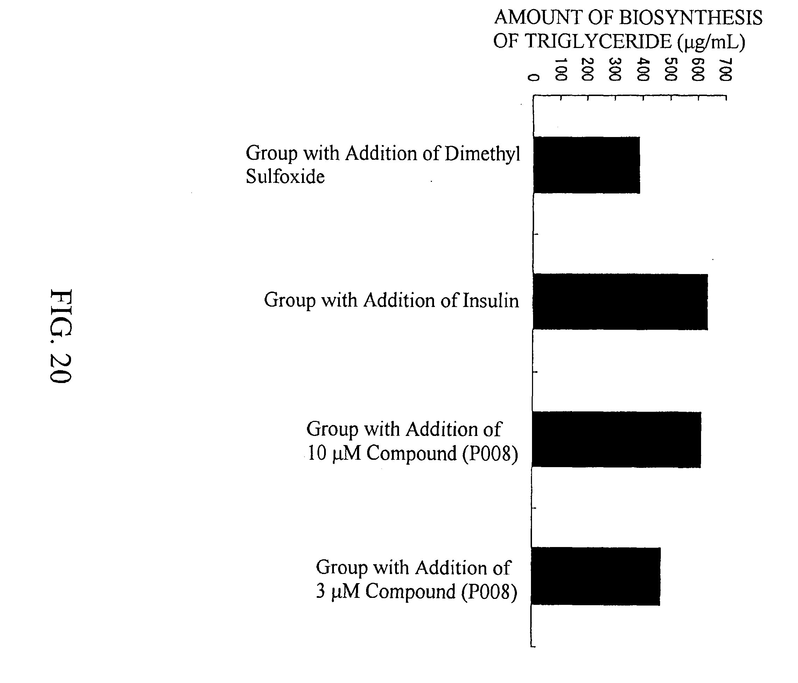

As a result, induction of biosynthesis of triglyceride was found in the group with addition of the compound (P008). In other words, an inducing action on differentiation into mature adipocytes was found in the compound (P008). The results are shown in Figure 20. In Figure 20, the axis of abscissas is each sample, and the axis of ordinates is the amount of biosynthesis of triglyceride (µg/mL).

### Example 21 Synergistic Enhancing Action by Polygodial and Insulin on Glucose Uptake

The amount of 2-deoxyglucose uptake into mature adipocytes stimulated with the sample in the adipocytes was determined partly in accordance with the method described in Example 1 as the evaluation of synergistic enhancing action by a low-concentration polygodial and a low-concentration insulin on glucose uptake.

The mature adipocytes were prepared according to the method described in Example 1.

After the termination of the culture, the medium was removed, and the cells were washed twice with a Dulbecco's modified Eagle's medium containing 0.1% (w/v) bovine serum albumin. Thereafter, the cells were cultured at 37°C overnight in the presence of 5% carbonic dioxide gas. After the overnight culture, the cells were washed twice with a HEPES buffered saline (140 mM NaCl, 5 mM KCl, 2.5 mM MgSO₄, 1 mM CaCl₂, 20 mM HEPES-Na (pH 7.4)). The amount 0.9 mL of the same buffer was added thereto. The cells were cultured at 37°C for 45 minutes. Subsequently, polygodial was added thereto so as to have a final concentration of 300 nM, and insulin was added thereto so as to have a final concentration of 0.05 µg/mL. The cells were further cultured for 30 minutes. During the culture, as control, there were set a group with addition of insulin so as to have a final concentration of 0.05 µg/mL to a well without addition of polygodial, and a group with addition of polygodial so as to have a final concentration of 300 nM to a well without addition of insulin. In addition, a group without addition of the sample was used as a negative control. Thereafter, the amount of 2-deoxy-[1,2-³H(N)]-glucose taken into the adipocytes was determined in the same manner as in the method described in Example 1.

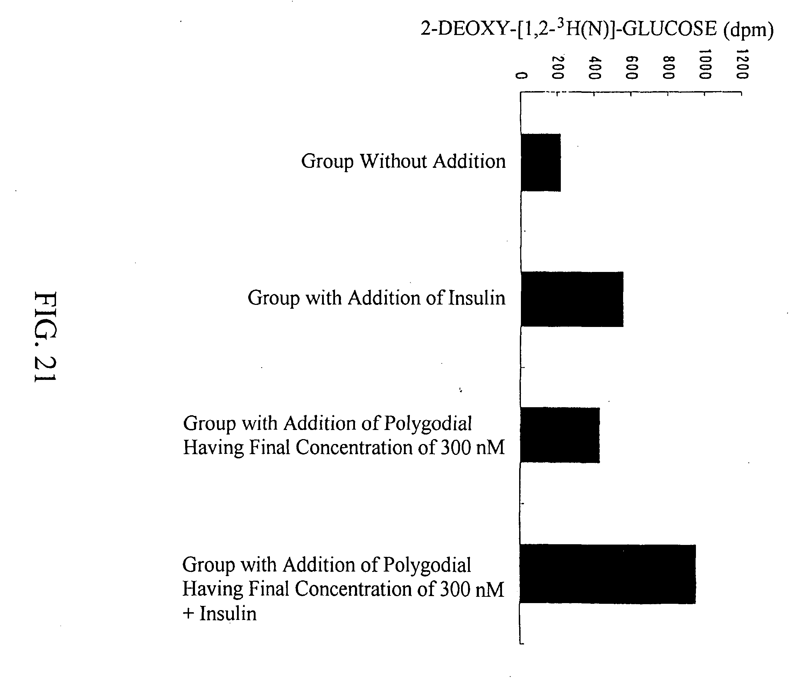

As a result, enhancement of 2-deoxy-[1,2-³H(N)]-glucose uptake was found in the group with addition of polygodial as well as in the group with addition of insulin as compared to the negative control. Enhancement of glucose uptake was found in the group with addition simultaneously with insulin more than any of the group with addition of insulin alone and the group with addition of polygodial alone. In other words, the enhancing action on glucose uptake was found to synergistically increase by simultaneously adding polygodial and insulin. The results are shown in Figure 21. In Figure 21, the axis of abscissas is each sample, and the axis of ordinates is the amount of 2-deoxy-[1,2-³H(N)]-glucose (dpm).

### Example 22 Enhancing Action by Polygodial on Glucose Uptake Inhibited by Cytochalasin B

The influence of cytochalasin B on 2-deoxyglucose uptake in mature adipocytes stimulated with the sample in the adipocytes was tested in accordance with the method described in Example 1 on whether or not the enhancing action by polygodial on glucose uptake shown in Example 1 is inhibited by cytochalasin B, which is an inhibitor of a glucose transporter.

Specifically, as a sample, there was set a group with addition of a dimethyl sulfoxide solution of polygodial so as to have a final concentration of 3 µM. There were set a group without addition of the sample as a negative control, and a group with addition of insulin so as to have a final concentration of 1 µg/mL as a positive control. Furthermore, in each group, there was set a group with addition of cytochalasin B (manufactured by Nacalai Tesque, Inc., 10435-81) so as to have a final concentration 40 µM at the same time as the time of addition of the sample and insulin. Thereafter, the amount of 2-deoxy-[1,2-³H(N)]-glucose taken into the adipocytes was determined in the same manner.

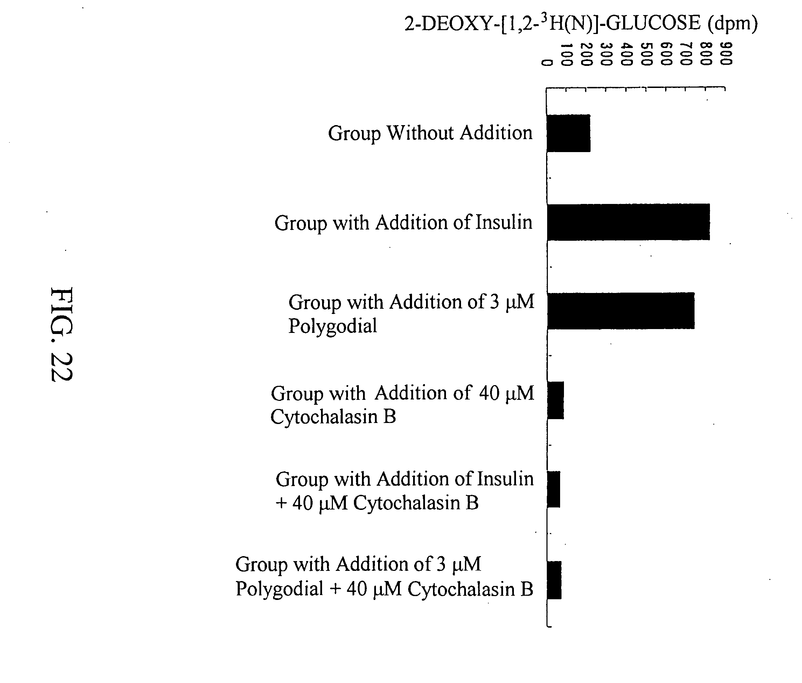

As a result, enhancement of 2-deoxy-[1,2-³H(N)]-glucose uptake was found in the group with addition of polygodial as well as in the group with addition of insulin, as compared to the negative control, but 2-deoxy-[1,2-³H(N)]-glucose uptake was almost completely inhibited by addition of cytochalasin B in every group. In other words, it could be confirmed that the enhancing action of polygodial on glucose uptake was mediated by a glucose transporter as well as in insulin. The results are shown in Figure 22. In Figure 22, the axis of abscissas is each sample, and the axis of ordinates is the amount of 2-deoxy-[1,2-³H(N)]-glucose (dpm).

### Example 23 Enhancing Action by Compound (P003) on Glucose Uptake

The amount of 2-deoxyglucose uptake into mature adipocytes stimulated with the sample in the adipocytes was determined in accordance with the method described in Example 1, except that the sample was added when each of the 0.1% (w/v) bovine serum albumin-containing Dulbecco's modified Eagle's medium or the HEPES buffered saline was exchanged, as the evaluation of enhancing action on glucose uptake and also as the evaluation of insulin-mimetic action of the compound (P003) prepared in Example 8.

Specifically, as a sample, there was set a group with addition of a dimethyl sulfoxide solution of the compound (P003) so as to have a final concentration of 30 µM or 10 µM. Here, there were set a group without addition of a sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

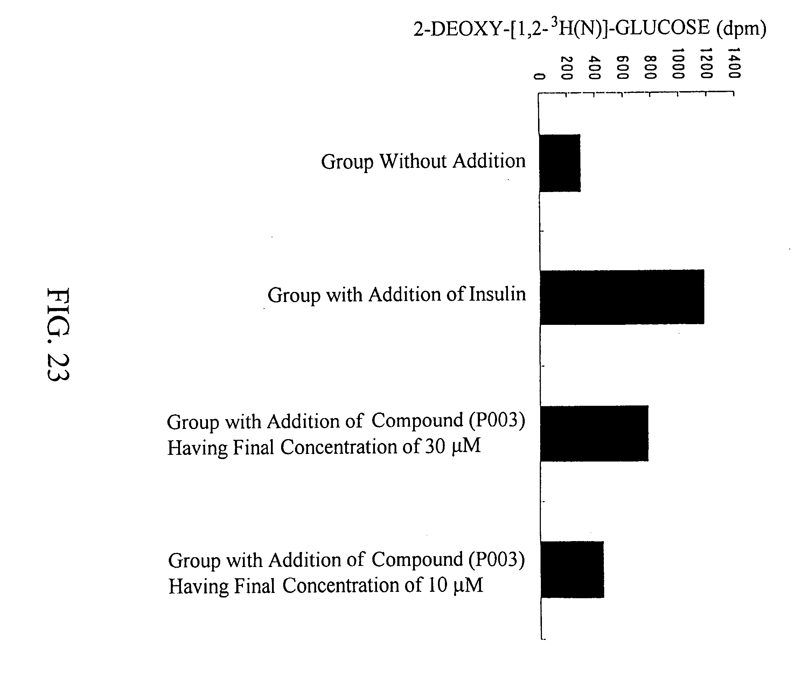

As a result, enhancement of 2-deoxy-[1,2-³H(N)]-glucose uptake was found in the group with addition of the compound (P003) as well as in the group with addition of insulin, as compared to the negative control. In other words, an enhancing action on glucose uptake was found in the compound (P003). The results are shown in Figure 23. In Figure 23, the axis of abscissas is each sample, and the axis of ordinates is the amount of 2-deoxy-[1,2-³H(N)]-glucose (dpm).

### Example 24 Enhancing Action by Isovelleral on Glucose Uptake

The amount of 2-deoxyglucose uptake into mature adipocytes stimulated with the sample in the adipocytes was determined in accordance with the method described in Example 1 as the evaluation of enhancing action on glucose uptake and also as the evaluation of insulin-mimetic action of isovelleral.

Specifically, as a sample, there was set a group with addition of a dimethyl sulfoxide solution of isovelleral so as to have a final concentration of 3 µM or 1 µM. Here, there were set a group without addition of a sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

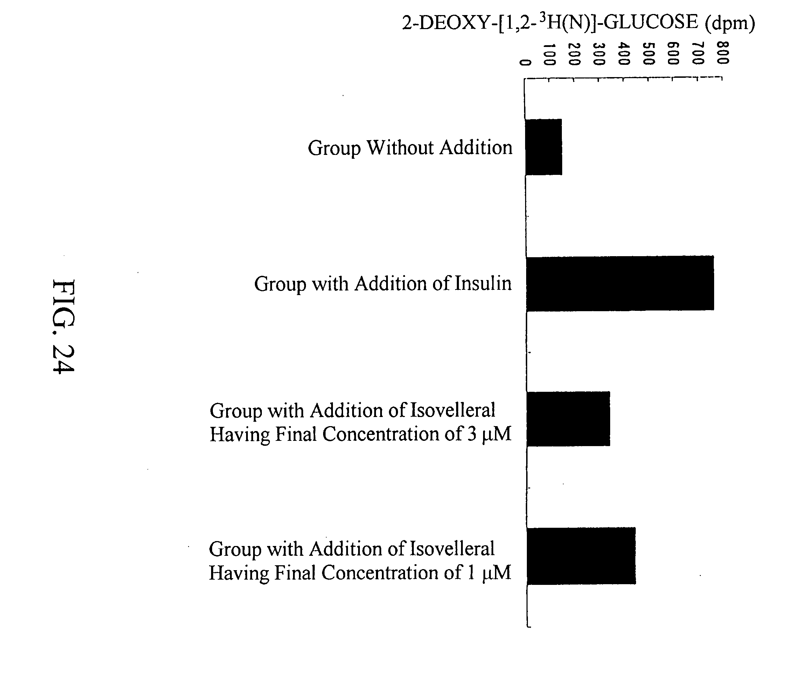

As a result, enhancement of 2-deoxy-[1,2-³H(N)]-glucose uptake was found in the group with addition of isovelleral as well as in the group with addition of insulin, as compared to the negative control. In other words, an enhancing action on glucose uptake was found in isovelleral. The results are shown in Figure 24. In Figure 24, the axis of abscissas is each sample, and the axis of ordinates is the amount of 2-deoxy-[1,2-³H(N)]-glucose (dpm).

### Example 25 Preparation of Epipolygodial

Polygodial was treated in tetrahydroxyfuran (THF) at 80°C for 2 hours in the presence of anhydrous potassium carbonate. The resulting reaction mixture was purified by silica chromatography, to give epipolygodial.

¹H-NMR (deuterated chloroform): δ0.93 (3H, s, -CH₃), 0.95 (3H, s, -CH₃), 0.98 (3H, s, -CH₃), 1.10-1.90 (7H, m), 2.24, 2.58 (2H, m, -CH₂-CH=), 3.28 (1H, m, -CH-CHO), 7.11 (1H, m, -CH₂-CH=), 9.43 (1H, s, -CHO), 9.87 (1H, d, J2 Hz, -CHO)

As the matrix, FAB-MS: m/z 234 (M+H)⁺ m-nitrobenzyl alcohol was used.

### Example 26 Enhancing Action by Epipolygodial on Glucose Uptake

The amount of 2-deoxyglucose uptake into mature adipocytes stimulated with the sample in the adipocytes was determined in accordance with the method described in Example 1 as the evaluation of enhancing action on glucose uptake and also as the evaluation of insulin-mimetic action of epipolygodial.

Specifically, as a sample, there was set a group with addition of a dimethyl sulfoxide solution of epipolygodial so as to have a final concentration of 3 µM, 1 µM or 0.3 µM. Here, there were set a group without addition of a sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

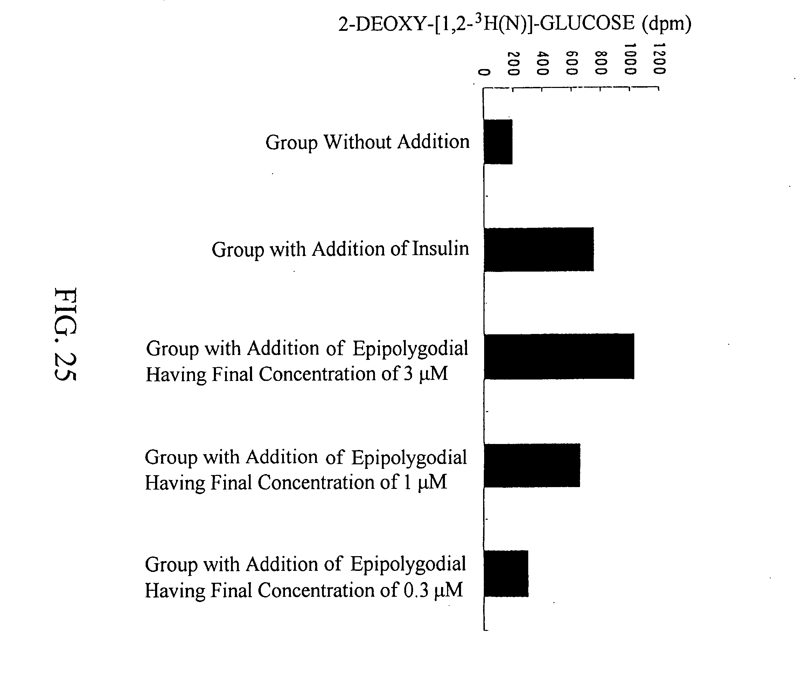

As a result, enhancement of 2-deoxy-[1,2-³H(N)]-glucose uptake was found in the group with addition of epipolygodial as well as in the group with addition of insulin, as compared to the negative control. In other words, an enhancing activity on glucose uptake was found in epipolygodial. The results are shown in Figure 25. In Figure 25, the axis of abscissas is each sample, and the axis of ordinates is the amount of 2-deoxy-[1,2-³H(N)]-glucose (dpm).

### Example 27 Amelioration Effects of Polygodial on Diabetes

Pathological amelioration of polygodial was examined using a type II diabetes model mice. Experiments were carried out with ten male 5-week old KK-Ay mice (CLEA Japan, Inc.) per group. Polygodial was dissolved in an olive oil so as to have a concentration of 0.03%, and the solution was forcibly orally administered to the mice once a day at 1 mg/kg weight for consecutive days. An olive oil was administered to each of mice in the control group at 3.3 mL/kg weight in the same manner. On the day before the beginning of administration and on the twenty-first day, blood was collected from the vein of mouse tails, and a blood glucose level was determined with a simple blood glucose meter, ACCU-CHEK Compact (manufactured by Roche Diagnostics K.K.). In addition, on the twenty-second day, blood was collected from the abdominal aorta, and the insulin concentration in serum was determined with Lebis Insulin Kit (manufactured by Shibayagi Co., Ltd.). The results are shown in Table 1. By administration of polygodial (1 mg/kg weight), the blood glucose level and the insulin concentration were found to be lowered. In other words, the action of ameliorating insulin resistance was found in polygodial. During the experimental period, no changes were found in weight and general symptoms.

**Table 1**

| | Blood Glucose Level (mg/dL) | | Insulin (ng/mL) |
|---|---|---|---|
| | Previous Day | Twenty-First Day | |
| Control Group | 329 ± 25 | 373 ± 43 | 21.4 ± 5 |
| Group Administered with Polygodial | 328 ± 25 | 231 ± 36 | 8.4 ± 4 |
| Mean ± Standard Error | | | |

### Example 28 Suppressive Action of Polygodial on Elevation in Blood Glucose Level After Glucose Tolerance

In order to examine a suppressive action of polygodial on elevation in the blood glucose level, a glucose tolerance test was conducted with five male 10-week old ICR mice (CLEA Japan, Inc.) per group. After fasting the mice for 18 hours, polygodial was dissolved in an olive oil so as to have a concentration of 0.3%, and the solution was forcibly orally administered to the mice at 10 mg/kg weight. An olive oil was administered to each of mice in the control group at 3.3 mL/kg weight in the same manner. After 1 hour from the administration, a 20% glucose solution was administered at 2 g/kg weight. Here, the glucose was administered in two ways, forcible oral administration and intraperitoneal administration. Before administration of the sample, before the glucose tolerance test, and after given periods of time after the glucose tolerance test, blood was collected from the vein of mouse tails, and a blood glucose level was determined. The results of the case where glucose was forcibly orally administered are shown in Table 2, and the results of the case where glucose was intraperitoneally administered are shown in Table 3. In both the administrations of glucose, a suppressive action by the administration of polygodial (10 mg/kg weight) on the elevation in the blood glucose level was found. In other words, efficacy as a therapeutic or prophylactic agent for diabetes was found in polygodial.

**Table 2**

| | Blood Glucose Level (mg/dL) | |
|---|---|---|
| | Control Group | Group Administered with Polygodial |
| Before Administration of Sample | 61 ± 4 | 94 ± 7 |
| Before Glucose Tolerance Test | 95 ± 7 | 126 ± 7 |
| 30 min. After Glucose Tolerance Test | 309 ± 21 | 260 ± 15 |
| 60 min. After Glucose Tolerance Test | 236 ± 18 | 224 ± 18 |
| 120 min. After Glucose Tolerance Test | 135 ± 8 | 138 ± 9 |

| | | |
|---|---|---|
| Glucose tolerance test was carried out by forcible oral administration. | | |
| Mean ± Standard Error | | |

**Table 3**

| | Blood Glucose Level (mg/dL) | |
|---|---|---|
| | Control Group | Group Administered with Polygodial |
| Before Administration of Sample | 79 ± 6 | 77 ± 4 |
| Before Glucose Tolerance Test | 114 ± 17 | 90 ± 7 |
| 30 min. After Glucose Tolerance Test | 298 ± 41 | 300 ± 56 |
| 60 min. After Glucose Tolerance Test | 321 ± 27 | 263 ± 34 |
| 120 min. After Glucose Tolerance Test | 172 ± 21 | 126 ± 10 |
| 180 min. After Glucose Tolerance Test | 115 ± 13 | 84 ± 5 |

| | | |
|---|---|---|
| Glucose tolerance test was carried out by intraperitoneal administration. | | |
| Mean ± Standard Error | | |

### INDUSTRIAL APPLICABILITY

According to the present invention, there is provided a medicament, a food, a beverage or a feed for the treatment or prevention of a disease accompanying an abnormality in an amount of insulin or insulin response, each comprising a compound having a specified structure. The medicament is useful as a therapeutic agent or prophylactic agent for a disease accompanying an abnormality in an amount of insulin or insulin response, such as diabetes or obesity. In addition, by taking the food or beverage as daily foodstuff, amelioration in symptoms and the like of a disease accompanying an abnormality in an amount of insulin or insulin response can be made. Therefore, the foodstuff of the present invention can be said to be functional foodstuff, and are useful in maintaining homeostasis of a living body due to its insulin-mimetic action. Also, according to the present invention, there is also provided an agent for an insulin-mimetic action comprising a compound having a specified structure. The agent for an insulin-mimetic action is useful in functional studies of insulin, or screening of a medicament for a disease associated with insulin. Also, according to the present invention, there is provided an agent for enhancing glucose uptake into a cell, comprising a compound having a specified structure. The agent for enhancing glucose uptake is also useful in treating or preventing a disease requiring the enhancing action on glucose uptake into a cell for the treatment or prevention, or preparing a food, beverage or feed for the treatment or prevention of the disease, or screening a drug for a disease requiring the enhancing action on glucose uptake. In addition, according to the present invention, there is provided an agent for inducing differentiation into an adipocyte, comprising a compound having a specified structure. The agent for inducing differentiation is also useful in treating or preventing a disease requiring the action for inducing differentiation into an adipocyte for the treatment or prevention, or preparing a food, beverage or feed for the treatment or prevention of the disease, or screening a drug for a disease requiring the action for inducing differentiation.

## Claims

1. A therapeutic agent or prophylactic agent for a disease accompanying an abnormality in an amount of insulin or insulin response, **characterized in that** the therapeutic agent or prophylactic agent comprises as an effective ingredient at least one compound selected from the group consisting of a compound represented by the following general formula (Formula 1): 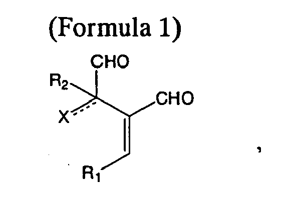
wherein a bond containing a dotted line is a single bond or a double bond; each of R₁ and R₂, which may be identical or different, is a hydrogen atom, a hydroxyl group which may be esterified or etherified, a halogen group, an acyl group, an amino group, a nitro group, a hydroperoxy group, an aliphatic group, an aromatic group, an aromatic aliphatic group, or a sugar residue; and X is a hydrogen atom or a carbon atom; in a case where X is a carbon atom, to the carbon atom may be added a hydrogen atom, a halogen group, an acyl group, an amino group, a nitro group, a hydroperoxy group, an aliphatic group, an aromatic group, an aromatic aliphatic group, or a sugar residue; or in a case where R₁ is an aliphatic group, X and R₁ may together form a 5- to 9-membered ring, and the 5- to 9-membered ring may have a resonance structure; a polycyclic structure comprising one or more 4- to 6-membered rings may be further formed adjacent to the 5- to 9-membered ring as long as a structure of a part excluding X and R₁ in the general formula (Formula 1) is maintained, wherein at least one substituent selected from a hydroxyl group which may be esterified or etherified, a halogen group, an acyl group, an amino group, a nitro group, a hydroperoxy group, an aliphatic group, an aromatic group, an aromatic aliphatic group and a sugar residue may be added to the polycyclic group; a ketone structure and/or an epoxy structure may be contained in the molecule; in a case where X and R₁ together form a 6-membered ring, if R₂ is a carbon atom, X and R₂ may further together form a 3-membered ring;
a compound represented by the following general formula (Formula 2): 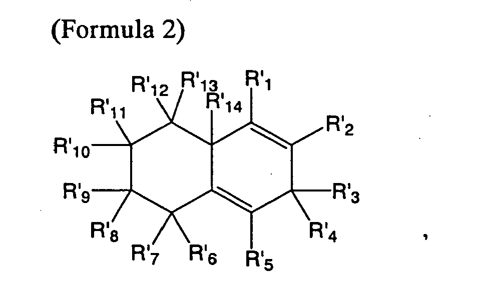
wherein each of R'₁ to R'₁₄, which may be identical or different, is a hydrogen atom, a halogen group, an acyl group, a hydroxyl group which may be esterified or etherified, an amino group, a nitro group, a hydroperoxy group, an aliphatic group, an aromatic group, an aromatic aliphatic group, or a sugar residue, or one or more pairs selected from R'₁ and R'₂, R'₂ and R'₃, R'₃ and R'₄, R'₄ and R'₅, R'₅ and R'₆, R'₆ and R'₇, R'₇ and R'₈, R'₈ and R'₉, R'₉ and R'₁₀, R'₁₀ and R'₁₁, R'₁₁ and R'₁₂, R'₁₂ and R'₁₃, R'₁₃ and R'₁₄, and R'₁₄ and R'₁ may form a ring containing one or more atoms selected from a carbon atom, an oxygen atom, a nitrogen atom and a sulfur atom in a possible range, wherein a hydroxyl group which may be esterified or etherified, a halogen group, an acyl group, an amino group, a nitro group, a hydroperoxy group, an aliphatic group, an aromatic group, an aromatic aliphatic group, or a sugar residue may be further bound to the above ring; or one or more pairs selected from R₃' and R'₄, R'₆ and R'₇, R'₈ and R'₉, R'₁₀ and R'₁₁, and R'₁₂ and R'₁₃ may together form a ketone structure with a carbon atom in the ring to which the pairs are bound;
a compound represented by the following general formula (Formula 3): 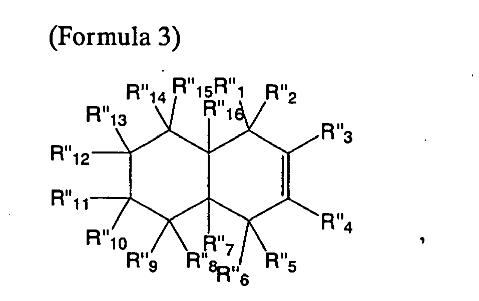
wherein each of R"₁ to R"₁₆, which may be identical or different, is a hydrogen atom, a halogen group, an acyl group, a hydroxyl group which may be esterified or etherified, an amino group, a nitro group, a hydroperoxy group, an aliphatic group, an aromatic group, an aromatic aliphatic group, or a sugar residue, or one or more pairs selected from R"₁ and R"₂, R"₂ and R"₃, R"₃ and R"₄, R"₄ and R"₅, R"₅ and R"₆, R"₆ and R"₇, R"₇ and R"₈, R"₈ and R"₉, R"₉ and R"₁₀, R"₁₀ and R"₁₁, R"₁₁ and R"₁₂, R"₁₂ and R"₁₃, R"₁₃ and R"₁₄, R"₁₄ and R"₁₅, R"₁₅ and R"₁₆, and R"₁₆ and R"₁ may form a ring containing one or more atoms selected from a carbon atom, an oxygen atom, a nitrogen atom and a sulfur atom in a possible range, wherein a halogen group, an acyl group, a hydroxyl group which may be esterified or etherified, an amino group, a nitro group, a hydroperoxy group, an aliphatic group, an aromatic group, an aromatic aliphatic group, or a sugar residue may be further bound to the above ring; or one or more pairs selected from R"₁ and R"₂, R"₅ and R"₆, R"₈ and R"₉, R"₁₀ and R"₁₁, R"₁₂ and R"₁₃, and R"₁₄ and R"₁₅ may together form a ketone structure with a carbon atom in the ring to which the pairs are bound;
derivatives thereof; and pharmacologically acceptable salts thereof.

2. The therapeutic agent or prophylactic agent according to claim 1, wherein the compound represented by the above-mentioned general formula (Formula 1) is at least one compound selected from the group consisting of a compound represented by the following general formula (Formula 4): 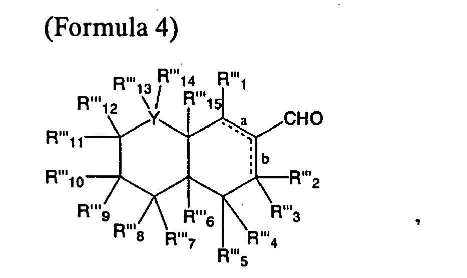
wherein Y is 0 or 1 carbon atom, and in a case where Y is 0 carbon atom, R'''₁₃ and R'''₁₄ are not present; each of R'''₁ and R'''₂, which is different from each other, is a hydrogen atom or an aldehyde group, and in a case where R'''₁ is an aldehyde group, a bond a is a single bond, and a bond b is a double bond; or in a case where R'''₂ is an aldehyde group, a bond a is a double bond, and a bond b is a single bond; each of R'''₃ to R'''₁₅, which may be identical or different, is a hydrogen atom, a halogen group, an acyl group, a hydroxyl group which may be esterified or etherified, or a saturated or unsaturated hydrocarbon group having 1 to 10 carbon atoms, with proviso that R'''₃ may not be present in some cases; or R'''₃ and R'''₄ may together form a 3-membered ring with a carbon atom in the ring in which these groups are bound to each other; or one or more pairs selected from R'''₄ and R'''₅, R'''₇ and R'''₈, and R'''₉ and R'''₁₀, R'''₁₁ and R'''₁₂, and R'''₁₃ and R"'₁₄ may together form a ketone structure and/or a 3-membered epoxy ring structure with a carbon atom in the ring to which the pairs are bound;
a compound represented by the following general formula (Formula 5): 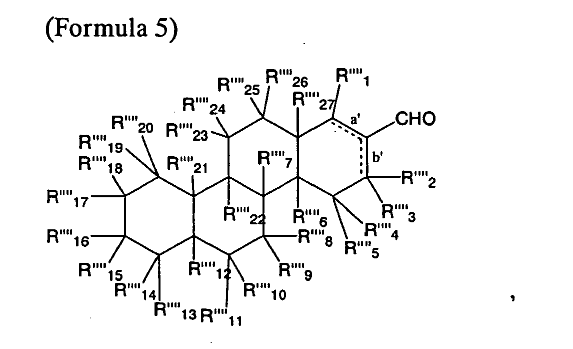
wherein each of R''''₁ and R''''₂, which is different from each other, is a hydrogen atom or an aldehyde group, and in a case where R''''₁ is an aldehyde group, a bond **a'** is a single bond, and a bond **b'** is a double bond; or in a case where R''''₂ is an aldehyde group, a bond **a'** is a double bond, and a bond **b'** is a single bond; each of R''''₃ to R''''₂₇, which may be identical or different, is a hydrogen atom, a halogen group, an acyl group, a hydroxyl group which may be esterified or etherified, or a saturated or unsaturated hydrocarbon group having 1 to 10 carbon atoms, with proviso that R''''₃ may not be present in some cases; or R''''₃ and R''''₄ may together form a 3-membered ring with a carbon atom in the ring in which these groups are bound to each other, or one or more pairs selected from R''''₄ and R''''₅, R''''₈ and R''''₉, R''''₁₀ and R''''₁₁, R''''₁₃ and R''''₁₄, R''''₁₅ and R''''₁₆, R''''₁₇ and R''''₁₈, R''''₁₉ and R''''₂₀, R''''₂₃ and R''''₂₄, and R''''₂₅ and R''''₂₆ may together form a ketone structure and/or form a 3-membered epoxy ring structure with a carbon atom in the above ring to which the pairs are bound; and a compound represented by the following general formula (Formula 6): 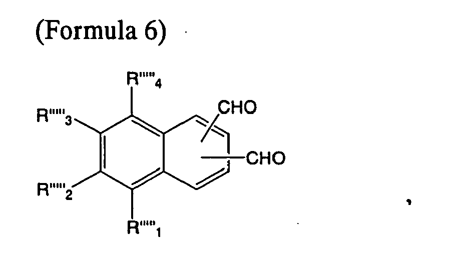
wherein each of R'''''₁ to R'''''₄, which may be identical or different, is a hydrogen atom, a halogen group, an acyl group, a hydroxyl group which may be esterified or etherified, or a saturated or unsaturated hydrocarbon group having 1 to 10 carbon atoms, wherein the two aldehyde groups attached to the ring on the right are added to adjacent carbon atoms in the ring.

3. The therapeutic agent or prophylactic agent according to claim 1, wherein in the compound represented by the above-mentioned general formula (Formula 2), each of R'₁, R'₂, R'₆, R'₇ and R'₁₄ in the compound is a methyl group, an aldehyde group, a carboxyl group, a methoxycarbonyl group, an acetoxymethyl group or a hydroxymethyl group, and wherein in the compound represented by the above-mentioned general formula (Formula 3), each of R"₁, R"₃, R"₈, R"₉ and R"₁₆ in the compound is a methyl group, an aldehyde group, a carboxyl group, a methoxycarbonyl group, an acetoxymethyl group or a hydroxymethyl group.

4. The therapeutic agent or prophylactic agent according to claim 1, wherein the compound represented by the above-mentioned general formula (Formula 2) is a compound represented by the following general formula (Formula 7): 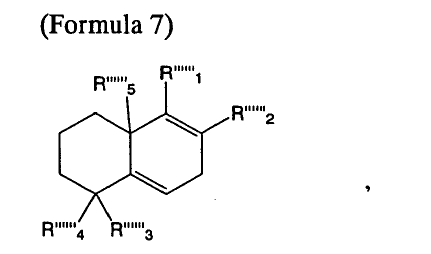
wherein each of R''''''₁ to R''''''₅, which may be identical or different, is a methyl group, an aldehyde group, a carboxyl group, a methoxycarbonyl group, an acetoxymethyl group, or a hydroxymethyl group.

5. The therapeutic agent or prophylactic agent according to claim 1, wherein the compound represented by the above-mentioned general formula (Formula 3) is a compound represented by the following general formula (Formula 8): 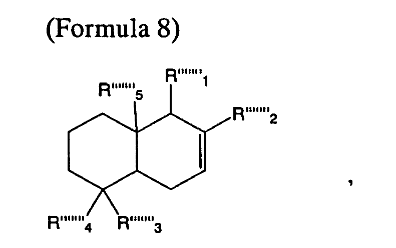
wherein each of R'''''''₁ to R'''''''₅, which may be identical or different, is a methyl group, an aldehyde group, a carboxyl group, a methoxycarbonyl group, an acetoxymethyl group, or a hydroxymethyl group.

6. The therapeutic agent or prophylactic agent according to claim 1, wherein the compound represented by the above-mentioned general formula (Formula 1) is at least one compound selected from the group consisting of polygodial, epipolygodial, isovelleral, 12-epi-scalaradial, naphthalene-2,3-dicarboxyaldehyde, and miogadial, and wherein the compound represented by the above-mentioned general formula (Formula 2) is at least one compound selected from the group consisting of dimethyl 3,5,6,7,8,8a-hexahydro-5,5,8a-trimethyl-1,2-naphthalenedicarboxylate, 3,5,6,7,8,8a-hexahydro-5,5,8a-trimethyl-1,2-naphthalenedimethanol, and 4,6,7,8,9,9a-hexahydro-6,6,9a-trimethyl-naphtho[1,2-c]furan-1(3H)-one, and wherein the compound represented by the above-mentioned general formula (Formula 3) is at least one compound selected from the group consisting of dimethyl 1,4,4a,5,6,7,8,8a-octahydro-5,5,8a-trimethyl-1,2-naphthalenedicarboxylate, 1,4,4a,5,6,7,8,8a-octahydro-5,5,8a-trimethyl-1,2-naphthalenedimethanol, 5,5a,6,7,8,9,9a,9b-octahydro-6,6,9a-trimethyl-naphtho[1,2-c]furan-3(1H)-one, 1-[(acetyloxy)methyl]-1,4,4a,5,6,7,8,8a-octahydro-5,5,8a-trimethyl-2-naphthalenecarboxyaldehyde, and 2-[(acetyloxy)methyl]-1,4,4a,5,6,7,8,8a-octahydro-5,5,8a-trimethyl-1-naphthalenecarboxyaldehyde.

7. An agent for an insulin-mimetic action, **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof and pharmacologically acceptable salts thereof, each as defined in claim 1.

8. The agent for an insulin-mimetic action according to claim 7, wherein the compound represented by the general formula (Formula 1) as defined in claim 1 is at least one compound selected from the group consisting of a compound represented by the general formula (Formula 4), a compound represented by the general formula (Formula 5), a compound represented by the general formula (Formula 6), each as defined in claim 2.

9. The agent for an insulin-mimetic action according to claim 7, wherein in the compound represented by the general formula (Formula 2) as defined in claim 1, each of R'₁, R'₂, R'₆, R'₇ and R'₁₄ is a methyl group, an aldehyde group, a carboxyl group, a methoxycarbonyl group, an acetoxymethyl group or a hydroxymethyl group, and wherein in the compound represented by the general formula (Formula 3) as defined in claim 1, each of R"₁, R"₃, R"₈, R"₉ and R"₁₆ is a methyl group, an aldehyde group, a carboxyl group, a methoxycarbonyl group, an acetoxymethyl group or a hydroxymethyl group.

10. The agent for an insulin-mimetic action according to claim 7, wherein the compound represented by the general formula (Formula 2) as defined in claim 1 is a compound represented by the general formula (Formula 7) as defined in claim 4.

11. The agent for an insulin-mimetic action according to claim 7, wherein the compound represented by the general formula (Formula 3) as defined in claim 1 is a compound represented by the general formula (Formula 8) as defined in claim 5.

12. The agent for an insulin-mimetic action according to claim 7, wherein the compound represented by the general formula (Formula 1) as defined in claim 1 is at least one compound selected from the group consisting of polygodial, epipolygodial, isovelleral, 12-epi-scalaradial, naphthalene-2,3-dicarboxyaldehyde, and miogadial, and wherein the compound represented by the general formula (Formula 2) as defined in claim 1 is at least one compound selected from the group consisting of dimethyl 3,5,6,7,8,8a-hexahydro-5,5,8a-trimethyl-1,2-naphthalenedicarboxylate, 3,5,6,7,8,8a-hexahydro-5,5,8a-trimethyl-1,2-naphthalenedimethanol, and 4,6,7,8,9,9a-hexahydro-6,6,9a-trimethyl-naphtho[1,2-c]furan-1(3H)-one, and wherein the compound represented by the general formula (Formula 3) as defined in claim 1 is at least one compound selected from the group consisting of dimethyl 1,4,4a,5,6,7,8,8a-octahydro-5,5,8a-trimethyl-1,2-naphthalenedicarboxylate, 1,4,4a,5,6,7,8,8a-octahydro-5,5,8a-trimethyl-1,2-naphthalenedimethanol, 5,5a,6,7,8,9,9a,9b-octahydro-6,6,9a-trimethyl-naphtho[1,2-c]furan-3(1H)-one, 1-[(acetyloxy)methyl]-1,4,4a,5,6,7,8,8a-octahydro-5,5,8a-trimethyl-2-naphthalenecarboxyaldehyde, and 2-[(acetyloxy)methyl]-1,4,4a,5,6,7,8,8a-octahydro-5,5,8a-trimethyl-1-naphthalenecarboxyaldehyde.

13. A food, beverage or feed for treating or preventing a disease accompanying an abnormality in an amount of insulin or insulin response, **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof and pharmacologically acceptable salts thereof, each as defined in claim 1.

14. The food, beverage or feed according to claim 13, wherein the compound represented by the general formula (Formula 1) as defined in claim 1 is at least one compound selected from the group consisting of a compound represented by the general formula (Formula 4), a compound represented by the general formula (Formula 5), a compound represented by the general formula (Formula 6), each as defined in claim 2.

15. The food, beverage or feed according to claim 13, wherein in the compound represented by the general formula (Formula 2) as defined in claim 1, each of R'₁, R'₂, R'₆, R'₇ and R'₁₄ is a methyl group, an aldehyde group, a carboxyl group, a methoxycarbonyl group, an acetoxymethyl group or a hydroxymethyl group, and wherein in the compound represented by the general formula (Formula 3) as defined in claim 1, each of R"₁, R"₃, R"₈, R"₉ and R"₁₆ is a methyl group, an aldehyde group, a carboxyl group, a methoxycarbonyl group, an acetoxymethyl group or a hydroxymethyl group.

16. The food, beverage or feed according to claim 13, wherein the compound represented by the general formula (Formula 2) as defined in claim 1 is a compound represented by the general formula (Formula 7) as defined in claim 4.

17. The food, beverage or feed according to claim 13, wherein the compound represented by the general formula (Formula 3) as defined in claim 1 is a compound represented by the general formula (Formula 8) as defined in claim 5.

18. The food, beverage or feed according to claim 13, wherein the compound represented by the general formula (Formula 1) as defined in claim 1 is at least one compound selected from the group consisting of polygodial, epipolygodial, isovelleral, 12-epi-scalaradial, naphthalene-2,3-dicarboxyaldehyde, and miogadial, and wherein the compound represented by the general formula (Formula 2) as defined in claim 1 is at least one compound selected from the group consisting of dimethyl 3,5,6,7,8,8a-hexahydro-5,5,8a-trimethyl-1,2-naphthalenedicarboxylate, 3,5,6,7,8,8a-hexahydro-5,5,8a-trimethyl-1,2-naphthalenedimethanol, and 4,6,7,8,9,9a-hexahydro-6,6,9a- trimethylnaphtho[1,2-c]furan-1(3H)-one, and wherein the compound represented by the general formula (Formula 3) as defined in claim 1 is at least one compound selected from the group consisting of dimethyl 1,4,4a,5,6,7,8,8a-octahydro-5,5,8a-trimethyl-1,2-naphthalenedicarboxylate, 1,4,4a,5,6,7,8,8a-octahydro-5,5,8a-trimethyl-1,2-naphthalenedimethanol, 5,5a,6,7,8,9,9a,9b-octahydro-6,6,9a-trimethyl-naphtho[1,2-c]furan-3(1H)-one, 1-[(acetyloxy)methyl]-1,4,4a,5,6,7,8,8a-octahydro-5,5,8a-trimethyl-2-naphthalenecarboxyaldehyde, and 2-[(acetyloxy)methyl]-1,4,4a,5,6,7,8,8a-octahydro-5,5,8a-trimethyl-1-naphthalenecarboxyaldehyde.

19. An agent for enhancing glucose uptake into a cell, **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof and pharmacologically acceptable salts thereof, each as defined in claim 1.

20. The agent for enhancing glucose uptake into a cell according to claim 19, wherein the compound represented by the general formula (Formula 1) as defined in claim 1 is at least one compound selected from the group consisting of a compound represented by the general formula (Formula 4), a compound represented by the general formula (Formula 5), a compound represented by the general formula (Formula 6), each as defined in claim 2.

21. The agent for enhancing glucose uptake into a cell according to claim 19, wherein in the compound represented by the general formula (Formula 2) as defined in claim 1, each of R'₁, R'₂, R'₆, R'₇ and R'₁₄ is a methyl group, an aldehyde group, a carboxyl group, a methoxycarbonyl group, an acetoxymethyl group or a hydroxymethyl group, and wherein in the compound represented by the general formula (Formula 3) as defined in claim 1, each of R"₁, R"₃, R"₈, R"₉ and R"₁₆ is a methyl group, an aldehyde group, a carboxyl group, a methoxycarbonyl group, an acetoxymethyl group or a hydroxymethyl group.

22. The agent for enhancing glucose uptake into a cell according to claim 19, wherein the compound represented by the general formula (Formula 2) as defined in claim 1 is a compound represented by the general formula (Formula 7) as defined in claim 4.

23. The agent for enhancing glucose uptake into a cell according to claim 19, wherein the compound represented by the general formula (Formula 3) as defined in claim 1 is a compound represented by the general formula (Formula 8) as defined in claim 5.

24. The agent for enhancing glucose uptake into a cell according to claim 19, wherein the compound represented by the general formula (Formula 1) as defined in claim 1 is at least one compound selected from the group consisting of polygodial, epipolygodial, isovelleral, 12-epi-scalaradial, naphthalene-2,3-dicarboxyaldehyde, and miogadial, and wherein the compound represented by the general formula (Formula 2) as defined in claim 1 is at least one compound selected from the group consisting of dimethyl 3,5,6,7,8,8a-hexahydro-5,5,8a-trimethyl-1,2-naphthalenedicarboxylate, 3,5,6,7,8,8a-hexahydro-5,5,8a-trimethyl-1,2-naphthalenedimethanol, and 4,6,7,8,9,9a-hexahydro-6,6,9a-trimethyl-naphtho[1,2-c]furan-1(3H)-one, and wherein the compound represented by the general formula (Formula 3) as defined in claim 1 is at least one compound selected from the group consisting of dimethyl 1,4,4a,5,6,7,8,8a-octahydro-5,5,8a-trimethyl-1,2-naphthalenedicarboxylate, 1,4,4a,5,6,7,8,8a-octahydro-5,5,8a-trimethyl-1,2-naphthalenedimethanol, 5,5a,6,7,8,9,9a,9b-octahydro-6,6,9a-trimethyl-naphtho[1,2-c]furan-3(1H)-one, 1-[(acetyloxy)methyl]-1,4,4a,5,6,7,8,8a-octahydro-5,5,8a-trimethyl-2-naphthalenecarboxyaldehyde, and 2-[(acetyloxy)methyl]-1,4,4a,5,6,7,8,8a-octahydro-5,5,8a-trimethyl-1-naphthalenecarboxyaldehyde.

25. An agent for inducing differentiation into an adipocyte, **characterized in that** the agent comprises as an effective ingredient at least one compound selected from the group consisting of a compound represented by the general formula (Formula 1), a compound represented by the general formula (Formula 2), a compound represented by the general formula (Formula 3), derivatives thereof and pharmacologically acceptable salts thereof, each as defined in claim 1.

26. The agent for inducing differentiation into an adipocyte according to claim 25, wherein the compound represented by the general formula (Formula 1) as defined in claim 1 is at least one compound selected from the group consisting of a compound represented by the general formula (Formula 4), a compound represented by the general formula (Formula 5), a compound represented by the general formula (Formula 6), each as defined in claim 2.

27. The agent for inducing differentiation into an adipocyte according to claim 25, wherein in the compound represented by the general formula (Formula 2) as defined in claim 1, each of R'₁, R'₂, R'₆, R'₇ and R'₁₄ is a methyl group, an aldehyde group, a carboxyl group, a methoxycarbonyl group, an acetoxymethyl group or a hydroxymethyl group, and wherein in the compound represented by the general formula (Formula 3) as defined in claim 1, each of R"₁, R"₃, R"₈, R"₉ and R"₁₆ is a methyl group, an aldehyde group, a carboxyl group, a methoxycarbonyl group, an acetoxymethyl group or a hydroxymethyl group.

28. The agent for inducing differentiation into an adipocyte according to claim 25, wherein the compound represented by the general formula (Formula 2) as defined in claim 1 is a compound represented by the general formula (Formula 7) as defined in claim 4.

29. The agent for inducing differentiation into an adipocyte according to claim 25, wherein the compound represented by the general formula (Formula 3) as defined in claim 1 is a compound represented by the general formula (Formula 8) as defined in claim 5.

30. The agent for inducing differentiation into an adipocyte according to claim 25, wherein the compound represented by the general formula (Formula 1) as defined in claim 1 is at least one compound selected from the group consisting of polygodial, epipolygodial, isovelleral, 12-epi-scalaradial, naphthalene-2,3-dicarboxyaldehyde, and miogadial, and wherein the compound represented by the general formula (Formula 2) as defined in claim 1 is at least one compound selected from the group consisting of dimethyl 3,5,6,7,8,8a-hexahydro-5,5,8a-trimethyl-1,2-naphthalenedicarboxylate, 3,5,6,7,8,8a-hexahydro-5,5,8a-trimethyl-1,2-naphthalenedimethanol, and 4,6,7,8,9,9a-hexahydro-6,6,9a-trimethyl-naphtho[1,2-c]furan-1(3H)-one, and wherein the compound represented by the general formula (Formula 3) as defined in claim 1 is at least one compound selected from the group consisting of dimethyl 1,4,4a,5,6,7,8,8a-octahydro-5,5,8a-trimethyl-1,2-naphthalenedicarboxylate, 1,4,4a,5,6,7,8,8a-octahydro-5,5,8a-trimethyl-1,2-naphthalenedimethanol, 5,5a,6,7,8,9,9a,9b-octahydro-6,6,9a-trimethyl-naphtho[1,2-c]furan-3(1H)-one, 1-[(acetyloxy)methyl]-1,4,4a,5,6,7,8,8a-octahydro-5,5,8a-trimethyl-2-naphthalenecarboxyaldehyde, and 2-[(acetyloxy)methyl]-1,4,4a,5,6,7,8,8a-octahydro-5,5,8a-trimethyl-1-naphthalenecarboxyaldehyde.

31. 3,5,6,7,8,8a-Hexahydro-5,5,8a-trimethyl-1,2-naphthalenedimethanol or a salt thereof.
